(19) Europäisches Patentamt
European Patent Office
Office européen des brevets

(11) Publication number: 0 412 821 B1

# (12) EUROPEAN PATENT SPECIFICATION

(45) Date of publication of patent specification: 09.11.94

(51) Int. Cl.[5]: C07D 498/10, A61K 31/42, //(C07D498/10,263:00,221:00)

(21) Application number: 90308779.9

(22) Date of filing: 09.08.90

The file contains technical information submitted after the application was filed and not included in this specification

## (54) 1-oxa-2-oxo-3,8-diazaspiro[4,5]decane derivatives, pharmaceutical compositions containing them and processes for preparing them.

(30) Priority: 10.08.89 HU 409489

(43) Date of publication of application: 13.02.91 Bulletin 91/07

(45) Publication of the grant of the patent: 09.11.94 Bulletin 94/45

(84) Designated Contracting States: AT BE CH DE DK ES FR GB GR IT LI LU NL SE

(56) References cited:
FR-A- 2 262 666
GB-A- 1 348 089

JOURNAL OF MEDICINAL CHEMISTRY, vol. 10, no. 4, 1967, pages 627-635,Washington, DC, US; M.A. DAVIS et al.: "New psychotropic agents. VIII. Analogs of amitriptyline containing the normeperidine group"

(73) Proprietor: RICHTER GEDEON VEGYESZETI GYAR R.T.
Gyömröi ut 19-21
H-1475 Budapest (HU)

(72) Inventor: Toth, Edit
Szabolcska M.u. 17
H-1114 Budapest (HU)
Inventor: Törley, Jozsef
Katona J.u. 41
H-1137 Budapest (HU)
Inventor: Görög, Sandor, Dr.
Somfa köz 13
H-1103 Budapest (HU)
Inventor: Szporny, Laszlo, Dr.
Szabolcska M.u. 7
H-1114 Budapest (HU)
Inventor: Kiss, Bela
Gergely u. 48
H-1103 Budapest (HU)
Inventor: Palosi, Eva, Dr.
Vend u. 21
H-1025 Budapest (HU)

EP 0 412 821 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHIMIE THERAPEUTIQUE, vol. 7, no. 6, 1972, pages 458-466, Paris, FR; J. MAILLARD et al.: "Composés cycloalcanespirohétérocycliques. VIII. Dérivés de (pipéridine-4' spiro) 5 oxazolidinones-2 (oxa-1 diaza-3,8 spiro [4,5] décanones-2)"**

**CHEMICAL ABSTRACTS, vol. 80, no. 15, 15th April 1974, pages 366-367, abstract no. 82582e, Columbus, Qhio, US; K. SINDELAR et al.: "Neurotropic and psychotropic agents. LXVII. 1-[4,4-Bis(4-fluorophenyl)butyl]-4-hydroxy-4-[-3-(trifluoromethyl)-4-chlorophenyl]piperidine and related compounds. New synthetic approaches"**

Inventor: **Groo, Dora, Dr.**
**Napraforgo u. 17**
**H-1021 Budapest (HU)**
Inventor: **Laszlovszky, Istvan, Dr.**
**Bartok B.u. 16**
**H-1111 Budapest (HU)**
Inventor: **Lapis, Erzsebet, Dr.**
**Abaliget u. 86**
**H-1173 Budapest (HU)**
Inventor: **Auth, Ferenc**
**Pacsirta u. 157/a**
**H-1024 Budapest (HU)**
Inventor: **Gaal, Laszlo, Dr.**
**Gergely u. 48**
**H-1103 Budapest (HU)**

(74) Representative: **Pett, Christopher Phineas et al**
**Frank B. Dehn & Co.**
**European Patent Attorneys**
**Imperial House**
**15-19 Kingsway**
**London WC2B 6UZ (GB)**

## Description

This invention relates to 1-oxa-2-oxo-3,8-diaza spiro[4,5]decane derivatives, pharmaceutical compositions containing them and processes for preparing them.

A number of therapeutically useful 2-oxo-1-oxa-3,8-diazaspiro[4,5]decane derivatives have been described in the literature, e.g. C.A. 71, 91359d (1969); C.A. 78, 23876q (1973); C.A. 78, 719688t; C.A. 81, 33153c and 105368b (1974); C.A. 95, 161765e (1981); as well as in the DE patent specifications Nos. 2,013,729, 2,013,668 and 2,163,000 and in the BE patent specifications Nos. 775,984, 774,170, 786,631 and 825,444; in the GB patent specification No. 1,100,281; in the published NL patent specification No. 7,214,689; as well as in the US patent specifications Nos. 3,555,033, 3,594,386, 4,244,961 and 4,255,432. Other 8-diazaspiro[4,5]decane derivatives have been described in C.A. 80, 82582e (1974).

We have now developed a novel range of compounds based on this ring system but for which the substituents bound in 4-position and optionally in 3-position of the spirodecane skeleton are substantially different.

According to one aspect of the present invention, there are provided 2-oxo-3,8-diazaspiro[4,5]decane derivatives of formula (I),

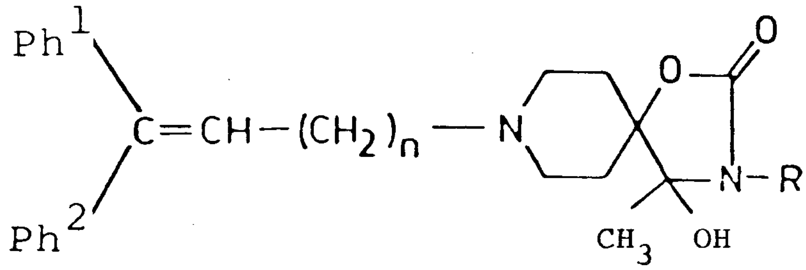

(I)

wherein

| | |
|---|---|
| R | represents a hydrogen atom or a $C_{1-12}$alkyl or $C_{3-6}$cycloalkyl group, or a carbocyclic $C_{6-10}$aryl or carbocyclic $C_{6-10}$aryl-$C_{1-4}$alkyl group optionally substituted on the aromatic ring by one or more halogen atoms, $C_{1-4}$alkyl or $C_{1-4}$alkoxy groups; |
| $Ph^1$ and $Ph^2$, | which may be the same or different, each represent a phenyl group unsubstituted or substitued by one or more halogen atoms or $C_{1-4}$alkyl, $C_{1-4}$alkoxy, trihalomethyl groups or hydroxyl groups optionally esterified by a $C_{1-4}$alkanoic acid; and |
| n | is 1 or 2, |

and optical isomers and mixtures thereof, and all solvates, hydrates, acid addition and quaternary ammonium salts thereof.

The compounds of the formula (I) may exist in various stereoisomeric forms such as geometrical isomers as well as racemates, separated optical isomers or their mixtures, all of which may also occur in the form of various solvates and hydrates. All these compounds and mixtures are also within the scope of the present invention.

According to another aspect of the invention, there is provided a process for the preparation of compounds of formula (I) as well as their acid addition and quaternary ammonium salts, which comprises

a) reacting a compound of formula (II)

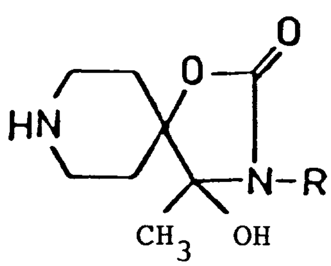

(II)

wherein R is as hereinbefore defined, with a compound of formula (III),

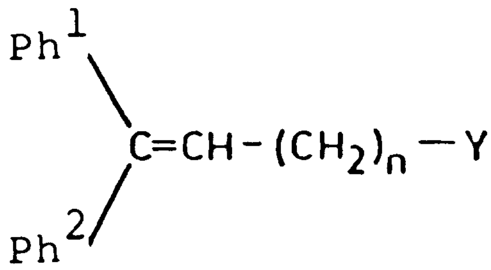

(III)

wherein $Ph^1$, $Ph^2$ and n are as hereinbefore defined and Y represents a halogen or a $C_{1-4}$alkylsulfonyloxy or arylsulfonyloxy group;

or

b) reacting a compound of formula (VII),

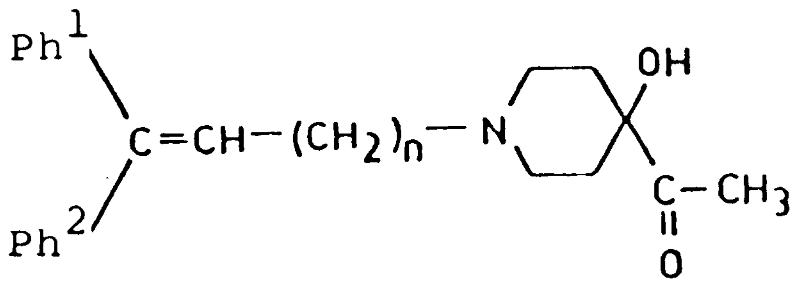

(VII)

wherein $Ph^1$, $Ph^2$ and n are as defined above , with an isocyanate of the formula R-NCO, wherein R is as defined above other than hydrogen, followed by cyclizing the resultant compound of formula (VIII),

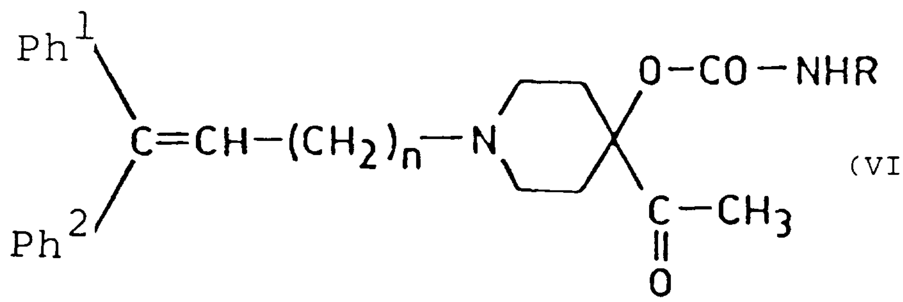

(VIII)

wherein R, $Ph^1$, $Ph^2$ and n are as defined above;

or

c) cyclizing a compound of formula (VIII) as defined above;

or

d) reacting a compound of formula (VI),

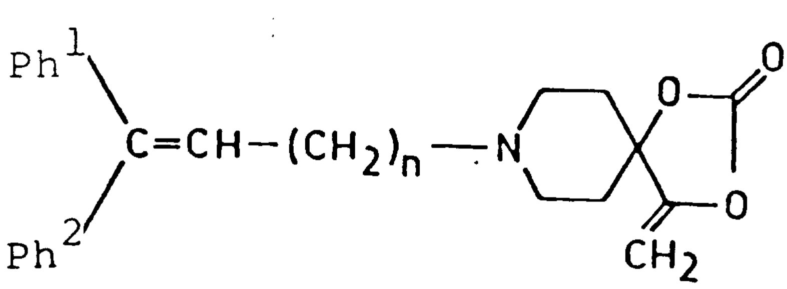

(VI)

wherein $Ph^1$, $Ph^2$ and n are as defined above, with an amine of formula $R\text{-}NH_2$, wherein R is as defined above;

or

e) hydrating a compound of formula (IV),

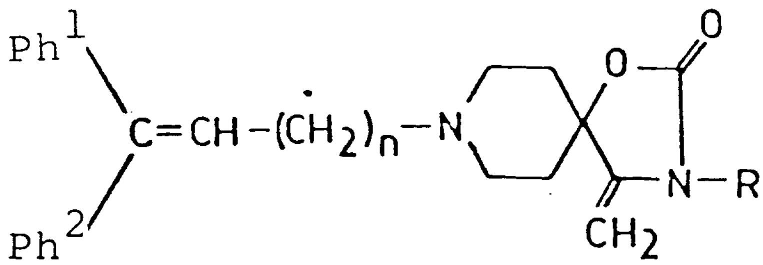

(IV)

wherein R, $Ph^1$, $Ph^2$ and n are as defined above, in an acidic medium;

followed if desired or necessary,

by transforming a functional group of a thus-obtained compound of formula (I) as defined above into another compound of formula (I) as hereinbefore defined in a known manner; and/or

reacting a thus-obtained compound of the formula (I) as defined above with an acid to give an acid addition salt and/or treating a compound of formula (I) as defined above, obtained as a salt, with a base to liberate the free basic form thereof and/or converting a thus-obtained compound of formula (I) as defined above into its quaternary ammonium salt.

In the process a) above, a 2-oxo-3,8-diazaspiro[4,5]decane derivative of the formula (II) is brought into reaction with a diphenylalkene derivative of the formula (III), wherein Y represents a leaving group, e.g. a mesyloxy-or tosyloxy group or a halogen atom, preferably chlorine or bromine. This reaction is preferably carried out in an inert organic solvent and preferably in the presence of a base capable of binding the acid liberated in the reaction. Suitable solvents include aliphatic alkanols such as ethanol, isopropanol or butanol; aromatic hydrocarbons such as chlorobenzene or toluene; ethers such as dibutyl ether or dioxane; tertiary aliphatic acid amides such as dimethylformamide or dimethylacetamide; ketones such as acetone, methyl ethyl ketone or methyl isobutyl ketone. Any mixture of the above solvents may also be employed. For binding the acid liberated in the reaction, inorganic or tertiary organic bases, e.g. carbonates or hydrogen carbonates of alkaline metals or alkaline earth metals as well as organic bases, e.g. triethylamine, dimethylaniline or pyridine may be used. An excess of the compound of the formula (II) is also suitable for this purpose. This reaction may be carried out between room temperature and the boiling point of the reaction mixture. Optionally, a catalyst may also be added. Suitable catalysts are e.g. alkaline metal iodides. It is preferable to carry out this reaction under an inert gas such as nitrogen or argon.

In the case of process b) above, a 4-acetyl-4-hydroxypiperidine derivative of the formula (VII) is reacted with an isocyanate of the formula R-NCO, then the thus-formed 4-acetyl-4-carbamoyloxypiperidine derivative of the formula (VIII) is cyclized. The condensation reaction according to the first step may be carried out in a known manner, e.g. [Houben-Weyl: Methoden der Organischen Chemie, Vol. VIII/3, page 137 to 147 (1952)]. The 4-acetyl-4-carbamoyloxy-piperidine derivative thus-obtained is preferably cyclized in the presence of a base. Alkaline metal acetates, carbonates, alkoxides, hydroxides and/or tertiary organic bases, e.g. pyridine, tripropylamine or picoline, may be used as basic catalysts in the cyclization. The organic bases may also serve as solvents for the reaction. Further suitable solvents include aliphatic alcohols such as methanol, ethanol, propanol or butanol; aliphatic, alicyclic or aromatic hydrocarbons such as methylene chloride, hexane, cyclohexane, benzene, toluene or xylene; acid amides such as dimethylformamide or N-methylpyrrolidone; ethers such as dibutyl ether or dioxan ; nitriles such as acetonitrile; sulfoxides, e.g. dimethyl sulfoxide; as well as any mixtures of the above solvents. The reaction may also be carried out without any solvent e.g. in a molten state. In order to accelerate the cyclization, the temperature may be suitably raised: the reaction is preferably accomplished between 40 °C and the boiling point of the reaction mixture. It is suitable to carry out this reaction under an inert gas such as argon or nitrogen. According to a preferred embodiment of this invention, the 4-acetyl-4-carbamoyloxypiperidine derivative of the formula (VIII) obtained from the reaction of 4-acetyl-4-hydroxypiperidine derivative of the formula (VII) with the isocyanate of the formula R-NCO is not isolated but cyclized directly in the same reaction mixture in the presence of a suitable base.

In the case of process c) above, the procedure as described for the second step of process b) above may be followed.

According to the process d) above, a compound of the formula (VI) is reacted with an amine of the formula $R-NH_2$. This reaction may be carried out in a suitable solvent or without any solvent. Suitable solvents include aliphatic, alicyclic or araliphatic alcohols such as ethanol, butanol, cyclohexanol, benzyl alcohol; aliphatic or aromatic hydrocarbons such as hexane, heptane, xylene, chlorobenzene or nitrobenzene; ethers, e.g. dioxan or di-n-butyl ether; and tertiary organic bases, e.g. picoline, triethylamine or pyridine. An excess of the amine of formula $R-NH_2$ may also serve as a solvent for the reaction. This procedure may be carried out at a temperature between room temperature and the boiling point of the reaction mixture, preferably under an inert gas, e.g. argon or nitrogen.

The hydration according to process e) above, is performed in a medium containing water, in the presence of mineral and/or organic acids. Suitable acids include the hydrogen halides, sulfuric acid, phosphoric acid, formic acid, aromatic sulfonic acid, oxalic acid and the like. This reaction is preferably carried out at a temperature between 5 °C and the boiling point of the reaction mixture.

If desired, the compounds of the formula (I) obtained as a result of the processes a) to e) may be transformed in a known manner into other compounds of formula (I) within the scope of the present invention.

If desired, the compounds of the formula (I) may be converted to the acid addition and quaternary ammonium salts by using know methods. For the preparation of acid addition salts inorganic or organic acids such as hydrogen halides, e.g. hydrochloric acid and hydrobromic acid; sulfuric acid, phosphoric acids as well as formic, acetic, propionic, oxalic, glycolic, maleic, fumaric, succinic, tartaric, ascorbinic, citric, malic, salicylic, lactic, benzoic, cinnamic, aspartic, glutamic, N-acetyl-aspartic or N-acetylglutamic acid as well as alkanesulfonic acids such as methanesulfonic acid or arenesulfonic acids, e.g. p-toluenesulfonic acid and the like, may be used.

Salt formation may be carried out e.g. in such a way that the relevant acid is added to the solution of the compound of the formula (I) prepared in an inert solvent, e.g. ethanol, and the salt formed is precipitated by adding preferably a water-immiscible organic solvent, e.g. diethyl ether.

For the preparation of quaternary ammonium salts a lower alkyl, alkenyl or benzyl halide or an alkyl sulfate may preferably be employed. The quaternization may be performed in an organic solvent such as acetone, acetonitrile, ethanol or their mixtures at a temperature in the range from room temperature up to the boiling point of the solvent.

The acid addition or quaternary ammonium salt obtained may be isolated e.g. by filtration and, when necessary, purified by recrystallization.

Conversely, the corresponding free bases can be liberated from their salts by an alkaline treatment.

The compounds of formula (III) may be prepared according to e.g. : Ber. 55, 3406 (1922); Ann. Chem. 555, 80 (1952); GB patent specification No. 683,950; Yakugaku Zasshi 82, 1088 (1952); J. Chem. Soc. 4066 (1959); Coll. Czechoslov. Chem. Commun. 38, 3879 (1973).

The preparation of Compounds of the formula (II) is described in our EP-A-0412820 of even date claiming priority from Hungarian patent application No. 4092/89.

The compounds of the formula (IV) can be prepared by reacting 2-oxo-3,8-diazaspiro[4,5]decane derivatives of the formula (XX)

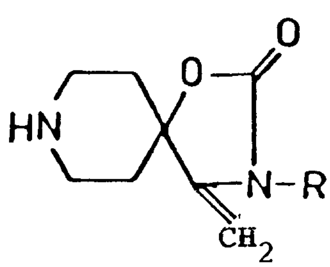

(XX)

wherein R is as defined above, with diphenylalkene derivatives of the formula (III), under conditions similar to those as discussed in process (a) herein. The preparation of compounds of formula (XX) is also described in our EP-A-0412820.

The compounds of the formula (VI) may be prepared by cyclizing a 4-carbamoyloxy-4-ethynylpiperidine derivative of the formula (V) in an acidic medium and then reacting the imino compound with water.

The carbamates of the formula (V)

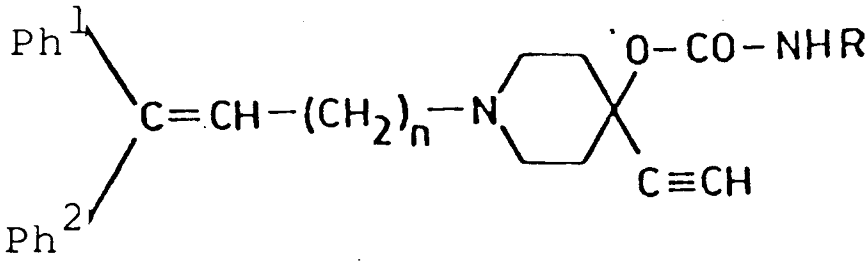

(V)

and (VIII) may be obtained by e.g. reacting a compound of the formula (IX),

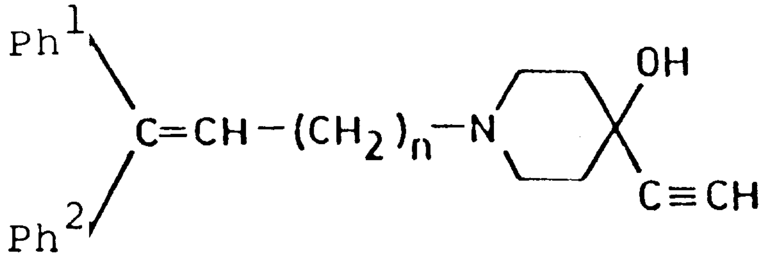

(IX)

or (VII), respectively, with an isocyanate of the formula R-NCO as described hereinabove.

The compounds of the formula (IX) may be prepared e.g. by the ethynylation reaction of suitably substituted 4-piperidone derivatives as described e.g. in the Hungarian patent specification No. 166,769 or in Farmaco (Pavia) Ed. Sci. 12, 34 (1957).

The 4-acetyl-4-hydroxypiperidine derivatives of the formula (VII) may be synthetized by e.g. hydrating a suitable 4-ethynyl-4-hydroxypiperidine derivative /see e.g.: Houben-Weyl: Methoden der Organischen Chemie, Vol. VII/2, pages 826 to 835 (1973)/ or by the alkaline treatment of a suitable 4-methylene-2-oxo-1,3-dioxa-8-azaspiro[4,5]decane derivative of the formula (VI).

The compounds of formula (I) according to the present invention and their salts possess valuable pharmacological properties. They exert e.g. a selective dopaminergic action in the central nervous system and show antihypoxic, antianoxic, antiischaemic and antiamnesic properties. Due to their dopaminergic effect, they inhibit the central dopamine (hereinafter: DA) receptors in the cortical and subcortical brain regions. They are capable of protecting against primary lessions caused by the cerebral hypoxia/ischaemia of various origin, e.g. the cerebral edema, memory damages induced by hypoxia and of prolonging the survival of mammals under severe hypoxic conditions. The compounds of the formula (I) may be widely used in the therapy for the prevention and treatment of various diseases such as mania, agitations of various origin, psychomotor disquiet, hyperkinesia, senile and multiinfarctual dementia, Alzheimer's disease, disturbances of the cognitive functions, ischaemic injuries and the like.

The pharmacological effects of the novel compounds of the formula (I) according to the present invention were studied by using the methods described hereinafter.

1. Inhibition of the apomorhine-induced locomotor hyperactivity and stereotypy

Apomorphine induces a characteristic syndrome in rats and various animal species which manifests itself in the hyperactivity and stereotypic behaviour of the animals /J. Pharm. Pharmacol. 19 627 (1957); J. Neurol. Transm. 40, 97 (1977); J. Psychiat. Res. 11, 1 (1974); J. Pharm. Pharmacol. 25, 1003 (1973); as well as Nature 263, 338 (1976)/.

Male Hannover-Wistar rats weighing 160 to 180 g were used in these tests. The test compounds were suspended in a 2% Tween 80 solution and diluted to the desired concentration by adding distilled water. The corresponding dose was administered to rats in a volume of 5 ml/kg. The control group was treated

with the above solution containing no test substance.

One hour following the oral treatment of a 2.5 mg/kg dose of the test compound, the rats were subcutaneously treated with 1 mg/kg of apomorphine hydrochloride.

15 minutes after administration of apomorphine, the animals were placed in a 5-channel behaviour-observing device controlled by a microprocessor and the coordinated and stereotypic motions of the animals were measured for 15 minutes. Chlozapine (chemically 8-chloro-11-(4-methyl-1-piperazinyl)-5H-dibenzo[b,e][1,4]diazepine) was used in a dose of 2.5 mg/kg as a reference drug. The results are given in the Table below as percentages of the control for both motion types.

The cataleptogenic (catalepsy-inducing) effect of the compounds was investigated by using the method of G. Stille and H. Launer [Arzneim.-Forsch. 21, 252 (1971)] . In these tests, male Wistar rats weighing 90 to 110 g were used and were orally treated with various doses of the test substances. The number of cataleptic animals was registered hourly for 6 hours following the treatment. An animal was considered to be cataleptic when it did not correct within 30 seconds its unusual body position caused by lifting its upper limbs onto a horizontal rod set at a height of 8 cm. The $ED_{50}$ value was calculated from the percentage of cataleptic animals. The results are summarized in Table 1 below.

The abbreviations used in the Table are as follows:

- LMA : locomotor activity
- n: number of animals
- p.o.: oral administration
- S.E.: standard error of the mean value
- A : 8-[4,4-bis(4-fluorophenyl)-3-butenyl]-3,4-dimethyl-4-hydroxy-2-oxo-1-oxa-3,8-diazaspiro[4,5]-decane hydrochloride
- B : 3-benzyl-8-[4,4-bis(4-fluorophenyl)-3-butenyl]-4-hydroxy-4-methyl-2-oxo-1-oxa-3,8-diazaspiro-[4,5]decane hydrochloride

Table 1

| Compound | Inhibition of apomorphine-induced LMA as percentage of the control | stereotypy | Cataleptogenic effect $ED_{50}$ mg/kg p.o. | n |
|---|---|---|---|---|
| A | -40 | 0 | >300 | 5 |
| B | -25 | +15 | >300 | 5 |
| Clozapine | -25 | +11 | 31.9 | 5 |

Control: LMA: 100% (438.9 ± 54.7 sec ± S.E.)

Stereotypy: 100% (105.0 ± 13.7 sec ± S.E.)

It is obvious from Table 1 that a 2.5 mg/kg oral dose of the compounds of formula (I) according to the present invention decreased the apomorphine-induced locomotor hyperactivity with the same or a significantly higher efficiency than the reference drug, whereas, similarly to clozapine, they do not inhibit the stereotypy. Their cataleptogenic effect was at least ten times as favourable as that of the reference drug. Thus, it can be expected that the extrapyramidal side effects of the compounds of the formula (I) according to this invention would be less frequent or even absent.

4. Inhibition of the cerebral (brain) edema

The cerebral edema-inhibiting effect of the compounds was investigated by the following method /Ann. Pharm. Fr. 42, 431 (1984)/.

A group consisting of 7 male Hannover-Wistar rats weighing 180 to 200 g were treated daily with 2.5 mg/kg of triethyl tin chloride (hereinafter TET) for 5 days to induce a cerebral edema. [The TET (Merck-Schuchardt, Darmstadt, FRG) was dissolved in distilled water under stirring and the animals were orally treated with the stirred solution at 7 a.m. daily].

The reference and test compounds were homogenized in a 0.5% by weight carboxymethylcellulose solution by using an Ultra Turrax stirrer and the stirring was continued during the administration thereof. These compounds were given orally first 1 hour, then 6 hours after the TET treatment in a dosage of 0.5 ml/100 g of body-weight in both cases. The control group received the solvent of TET (i.e. distilled water) and the carrier of the compounds (i.e. 0.5% by weight carboxymethylcellulose solution) at the same intervals and dosages as given above.

On the 5th day of treatment, 2 hours following the last administration of the test compound, the animals were decapitated, the whole brain was rapidly taken out, washed with cold, 0.9% by weight sodium chloride solution and the moisture was removed by filter paper. The wet weights of the brains were weighed with an accuracy of a tenth of a mg and placed onto an previously weighed aluminum foil. Subsequently, the brains were dried at 90 °C for 92 hours, then the total (gross) weight (i.e. the dry brain weight together with the weight of the aluminum foil) was determined. The water content of the brain and the change thereof, respectively, were calculated from the difference of the wet and dry brain weight. The change was expressed as percentage of the protection as follows:

$$\text{protection \%} = \frac{(\text{TET-Ko}) - (\text{TET-V})}{(\text{TET-Ko}) - (\text{Veh-Ko})} \times 100$$

wherein

(TET-Ko) means the mean water content (%) of the brain of animals treated with TET and carrier;

(TET-V) means the mean water content (%) of the brain of animals trated with TET and test compound;

(Veh-Ko) means the mean water content (%) of the brain of animals treated with distilled water and carrier.

The deviations of changes in the water content and dry weight of the brain were compared by using Student's "t" trial. Piracetam and dihydroergotoxine were used as reference drugs. The results are summarized in Table 4 below.

Abbreviations used in Table 4 are as follows:

TET: triethyl tin chloride

dw: distilled water

Veh: vehicle (0.5% by weight carboxy methylcellulose solution)

D : 8-[4,4-bis(4-fluorophenyl)-3-butenyl]-3-[2-(3,4-dimethoxyphenyl)ethyl]-4-hydroxy-4-methyl-2-oxo-1-oxa-3,8-diazaspiro-[4,5]decane maleate

E : 8-[4,4-bis(4-fluorophenyl)-3-butenyl]-4-hydroxy-4-methyl-2-oxo-1-oxa-3,8-diazaspiro[4,5]decane hydrochloride

Table 4

| Treatment | Dose μmol/kg | Protection % |
|---|---|---|
| dw + Veh | - | - |
| TET + Veh | - | - |
| TET + compound D | 100 | 102.2 |
| TET + compound E | 100 | 114.3 |
| TET + PIR | 100 | 16.7 |
| TET + DHE | 30[x] | 25.6 |

[x] DHE was administered in a dose of 30 mg/kg in the above experiment.

Based on the above investigations, the compounds according to the present invention appear capable of efficiently protecting animals from amnesia and memory damage developed due to hypoxia and of prolonging in low doses the survival time of the animals suffering from a severe hypoxia.

In the central nervous system a severe edema and increase in the brain water content as well as significant damages in the metabolizing activity of the brain are induced, and the respiration of cells, the oxidative phosphorylation, oxidation of glutamate, succinate and glucose and the like are inhibited, by

triethyl tin chloride. These severe alterations are observed also in hypoxia and ischaemia. These primarily appear as the development of a cerebral edema and its effects such as disturbances of the cognitive functions, dementia and the like. The compounds according to the invention provide protection from the development of a cerebral edema whereas no protective effect was achieved by the reference drugs.

The compounds according to the invention can be formulated into pharmaceutical compositions. These compositions may be administered orally, rectally and/or parenterally. For oral administration, the composition may be formulated e.g. as a tablet, dragée or capsule. In order to prepare oral compositions, e.g. lactose or starch may be used as carriers. Gelatine, sodium carboxymethylcellulose, methylcellulose, polyvinylpyrrolidone or starch gum are suitable binding or granulation agents. As disintegrating agents mainly potato starch or microcrystalline cellulose may be added though ultraamylopectin or formaldehyde-casein and the like are also useful. Talc, colloidal silicic acid, stearin, calcium or magnesium stearate and the like are suitable anti-adhesive and sliding agents. Liquid oral compositions can be formulated e.g. as suspensions, syrups or elixirs which may contain water, glycols, oils, alcohols as well as colouring and flavouring agents.

Tablets may be prepared e.g. by compression following wet granulation. The mixture of the active ingredient with the carriers and optionally with a part of the disintegrating additive is granulated with an aqueous, alcoholic or aqueous-alcoholic solution of the binding agents, then the granulate is dried. Subsequently, after mixing the other disintegrating, sliding and anti-adhesive additives to the dried granulate, the mixture is compressed into tablets. If desired, the tablets may be provided with a groove in order to facilitate their administration. Tablets may also directly be prepared from a mixture containing the active ingredient and suitable additives. The tablets may optionally be converted to dragées by employing commonly used pharmaceutical additives, e.g. protective, flavouring or colouring agents such as sugar, cellulose derivatives (methyl- or ethylcellulose, sodium carboxymethylcellulose and the like), polyvinylpyrrolidone, calcium phosphate, calcium carbonate, food dyes, dyeing lacquers, aromatizing agents, iron oxide, pigments and the like. Encapsulated compositions may be prepared by filling a mixture of the active ingredient with the additives into capsules.

For rectal administration, the composition of the invention may be formulated as a suppository containing a carrier mass, the so-called "adeps pro suppositorio", in addition to the active ingredient. As carriers, vegetable fats such as hardened vegetable oils, or triglycerides of $C_{12-18}$ fatty acids (preferably the carriers bearing the trade name Witepsol) may be used. The active ingredient is uniformly distributed in the molten carrier mass, then suppositories are prepared by moulding.

For parenteral administration, the composition of the invention may be formulated as an injectable solution. For preparing these injectable solutions, the active ingredients are dissolved in distilled water and/or various organic solvents, e.g. glycol ethers, if desired, in the presence of solubilizing agents such as polyoxyethylene sorbitan monolaurate or monooleate or monostearate (Tween 20, Tween 60 or Tween 80), respectively. The injectable solution may further contain various auxiliary agents, e.g. preservatives such as ethylenediamine tetraacetate as well as pH-modifying and buffering substances or, if desired, a local anaesthetic agent such as lidocaine. Before filling into the ampoules, the injectable solution containing the composition of the invention is filtered and after filling in, it is subjected to sterilization.

According to a further feature of this invention, the compounds of this invention as defined herein may be used for the manufacture of a medicament for the treatment of disturbances of the cognitive functions of mammals (including man). This treatment comprises administering a therapeutically effective amount of an active ingredient of the formula (I) or a pharmaceutically acceptable acid addition salt or quaternary ammonium salt thereof to the patient. Depending on the type and severity of the disease and conditions of the patient, the daily dose may vary between 0.1 and 40 mg/kg which may be administered once daily or in several subdoses via an oral, rectal or parenteral route.

The invention is illustrated by way of the following Preparations and Examples.

Preparation 1

4-methylene-2-oxa-3-n-propyl-1-oxa-3,8 diazaspiro [4,5] decane

(a) 8-benzyl-3-n-butyl-4-methylene-2-oxo-1-oxa-3,8-diazaspiro[4,5]decane

21.5 g of 1-benzyl-4-ethynyl-4-hydroxypiperidine are boiled under reflux with 12.9 g of n-butyl isocyanate in the presence of 0.4 g of anhydrous potassium acetate in 66 ml of 2-picoline under nitrogen for 6 hours. After evaporating 2-picoline under reduced pressure and dissolving the residue in benzene, the organic solution is washed with water and dried over anhydrous magnesium sulfate. After filtration on an

aluminum oxide layer the benzene solution is evaporated under reduced pressure. The crude product obtained is recrystallized from n-heptane to give the pure title substance in 78.5% yield, m.p.: 57-58 °C.
Analysis:

| Calculated for $C_{19}H_{26}N_2O_2$ | | | |
|---|---|---|---|
| | C 72.58; | H 8.33; | N 8.91%; |
| found: | C 72.55; | H 8.53; | N 9.06%. |

Using the appropriate starting materials the following compounds may be prepared in an analogous manner;

8-Formyl-4-methylene-2-oxo-3-phenyl-1-oxa-3,8-diazaspiro[4,5]decane, m.p.: 171-172 °C;

8-Benzyloxycarbonyl-4-methylene-2-oxo-3-phenyl-1-oxa-3,8-diazaspiro[4,5]decane, m.p.: 145-146 °C;

4-Methylene-2-oxo-3-phenoxycarbonyl-3-phenyl-1-oxa-3,8-diazaspiro[4,5]decane, m.p.: 208-210 °C;

8-Benzyl-3-cyclohexyl-4-methylene-2-oxo-1-oxa-3,8-diazaspiro[4,5]decane, m.p.: 128-130 °C (the hydrogen fumarate salt melts at 207-208 °C);

8-Benzyl-3-ethyl-4-methylene-2-oxo-1-oxa-3,8-diazaspiro[4,5]decane, m.p.: 103-104 °C (hydrogen maleate salt m.p.: 184-186 °C);

8-Benzyl-3-tert-butyl-4-methylene-2-oxo-1-oxa-3,8-diazaspiro[4,5]decane, m.p.: 116-117 (dihydrogen citrate salt m.p.: 132-133 °C);

8-Benzyl-4-methylene-2-oxo-3-phenyl-1-oxa-3,8-diazaspiro[4,5]decane, m.p.: 134-135 °C;

8-Benzyl-3-isopropyl-4-methylene-2-oxo-1-oxa-3,8-diazaspiro[4,5]decane, m.p.: 96-97 °C;

8-Benzyl-3-methyl-4-methylene-2-oxo-1-oxa-3,8-diazaspiro[4,5]decane hydrogen maleate, m.p.: 210-211 °C;

8-Benzyl-4-methylene-2-oxo-3-propyl-1-oxa-3,8-diazaspiro[4,5]decane dihydrogen citrate, m.p.: 168-171 °C;

(b) 3-tert-butyl-4-methylene-2-oxo-8-phenoxycarbonyl-1-oxa-3,8-diazaspiro[4,5]decane

A solution of 3.6 g of phenyl chloroformate in 5 ml of methylene chloride are dropped into a solution of 6.3 g of 8-benzyl-3-tert-butyl-4-methylene-2-oxo-1-oxa-3,8-diazaspiro[4,5]decane in 30 ml of methylene chloride under argon at 0 °C while stirring, then the reaction mixture is stirred at room temperature for one additional hour. After termination of the reaction the mixture is diluted with 35 ml of methylene chloride, extracted with 4 N sodium hydroxide solution and washed to neutral with water. After drying over anhydrous magnesium sulfate the solvent is evaporated under reduced pressure. After adding n-hexane to the residue the solid precipitate is filtered off and recrystallized from isopropanol to obtain the title substance in 82% yield, m.p.: 125-126 °C.
Analysis:

| Calculated for $C_{19}H_{24}N_2O_4$ | | | |
|---|---|---|---|
| | C 66.26; | H 7.02; | N 8.13%; |
| found: | C 66.33; | H 7.10; | N 8.10%. |

Using the appropriate starting materials the following compounds may be prepared in an analogous manner.

3-Benzyl-8-benzyloxycarbonyl-4-methylene-2-oxo-1-oxa-3,8-diazaspiro[4,5]decane, oil;

8-benzyloxycarbonyl-3-butyl-4-methylene-2-oxo-1-oxa-3,8-diazaspiro[4,5]decane, m.p.: 47-48 °C;

8-ethoxycarbonyl-3-methyl-4-methylene-2-oxo-1-oxa-3,8-diazaspiro[4,5]decane, m.p.: 121-122 °C;

3-(3,4-dichlorophenyl)-4-methylene-2-oxo-8-phenoxycarbonyl-1-oxa-3,8-diazaspiro[4,5]decane, m.p.: 220-222 °C;

3-methyl-4-methylene-2-oxo-8-phenoxycarbonyl-1-oxa-3,8-diazaspiro[4,5]decane, m.p.: 118-119 °C; and

4-methylene-2-oxo-8-phenoxycarbonyl-3-propyl-1-oxa-3,8-diazaspiro[4,5]decane, m.p.: 96-98 °C;

(c) 4-methylene-2-oxo-3-n-propyl-1-oxa-3,8-diazaspiro[4,5]decane

A suspension containing 0.5 g of 10% by weight palladium-on-carcoal catalyst in 5 ml of water is added to the solution of 5.0 g of 8-benzyloxycarbonyl-4-methylene-2-oxo-3-n-propyl-1-oxa-3,8-diazaspiro[4,5]-decane in 45 ml of methanol at 0 °C under argon while stirring. To this mixture 1 ml of 45.8% aqueous hydrazine solution is introduced and the reaction mixture is refluxed for 10 to 15 minutes. After cooling down to room temperature and filtering off the catalyst the solvent is evaporated under reduced pressure and the crude evaporation residue is recrystallized from benzene to give the title compound in 95% yield, m.p.: 96-97 °C.

Using the appropriate starting materials the following compounds may be prepared in an analogous manner;
3-Ethyl-4-methylene-2-oxo-1-oxa-3,8-diazaspiro[4,5]decane, m.p.: 106-108 °C;
3-isopropyl-4-methylene-2-oxo-1-oxa-3,8-diazaspiro[4,5]decane, m.p.: 151-152 °C;
4-methylene-3-(1-naphthyl)-2-oxo-1-oxa-3,8-diazaspiro[4,5]decane, m.p.: 208-209 °C;
3-butyl-4-methylene-2-oxo-1-oxa-3,8-diazaspiro[4,5]decane, oil;
4-methylene-2-oxo-3-phenyl-1-oxa-3,8-diazaspiro[4,5]decane, m.p.: 185-186 °C;
3-tert-Butyl-4-methylene-2-oxo-1-oxa-3,8-diazaspiro[4,5]decane, m.p.: 138-139 °C;
3-heptyl-4-hydroxy-4-methyl-2-oxo-1-oxa-3,8-diazaspiro[4,5]decane, m.p.: 139-140 °C;
3-[2-(3,4-dimethoxyphenyl)ethyl]-4-hydroxy-4-methyl-2-oxo-1-oxa-3,8-diazaspiro[4,5]decane, m.p.: 190-191 °C;
3-benzyl-4-methylene-2-oxo-1-oxa-3,8-diazaspiro[4,5]decane, m.p.: 77-79 °C;
3-heptyl-4-methylene-2-oxo-1-oxa-3,8-diazaspiro[4,5]decane, oil;
3-decyl-4-methylene-2-oxo-1-oxa-3,8-diazaspiro[4,5]decane, oil;
3-cyclohexyl-4-methylene-2-oxo-1-oxa-3,8-diazaspiro[4,5]decane,m.p.: 141-142 °C;
3-[2-(3,4-dimethoxyphenyl)ethyl]-4-methylene-2-oxo-1-oxa-3,8-diazaspiro[4,5]decane, m.p.: 107-109 °C.

Preparation 2

4-hydroxy-4-methyl-2-oxo-1-oxa-3,8-diazaspiro[4,5]decane

A solution containing 4.2 g of 8-benzyl-4-hydroxy-4-methyl-2-oxo-1-oxa-3,8-diazaspiro[4,5]decane in 42 ml of 95% methanol is refluxed in the presence of 0.75 g of hydrazine hydrate and 0.42 g of 10% by weight palladium-on-charcoal catalyst under vigorous stirring for 30 minutes. After cooling the reaction mixture to room temperature the catalyst is filtered off, washed with methanol and after combining the filtrate with the washings, the solution is evaporated under reduced pressure. After thoroughly triturating the residue with acetone the crystalline product obtained is filtered off and dried to give the title compound in 9.5% yield, m.p.: 192-194 °C.
Analysis:

| Calculated for $C_8H_{14}N_2O_3$ | | | |
|---|---|---|---|
| | C 51.60; | H 7.58; | N 15.04%; |
| found: | C 51.58; | H 7.55; | N 15.2%. |

Preparation 3

8-[4,4-bis(4-fluorophenyl)-3-butenyl]-3-ethyl-4-methylene-2-oxo-1-oxa-3,8-diazaspiro[4,5]decane

A mixture containing 7.8 g of 3-ethyl-4-methylene-2-oxo-1-oxa-3,8-diazaspiro[4,5]decane, 8.5 ml of anhydrous triethylamine, 80 ml of anhydrous methyl ethyl ketone, 0.3 g of potassium iodide and 16.7 g of 4,4-bis(4-fluorophenyl)-3-butenyl chloride is refluxed under nitrogen while stirring for 15 hours, then the solvent is distilled off under reduced pressure. After adding benzene and water to the residue, the benzene layer is washed with water to neutral, dried over anhydrous magnesium sulfate, filtered through an aluminum oxide bed and evaporated under reduced pressure. After recrystallizing the residue from ethanol, the title product is obtained in 68.7% yield, m.p.: 138-139 °C.
Analysis:

| Calculated for $C_{26}H_{28}F_2N_2O_2$ | | | | |
|---|---|---|---|---|
| | C 71.21; | H 6.44; | F 8.66; | N 6.39%; |
| found: | C 71.32; | H 6.46; | F 8.75; | N 6.58%. |

Example 1

8-[3,3-bis(4-fluorophenyl)-2-propenyl]-4-hydroxy-4-methyl-2-oxo-3-n-propyl-1-oxa-3,8-diazaspiro[4,5]decane

9.3 g of 3,3-bis(4-fluorophenyl)-2-propenyl bromide dissolved in 50 ml of acetone are added portionwise to a mixture containing 6.85 g of 4-hydroxy-4-methyl-2-oxo-3-n-propyl-1-oxa-3,8-diazaspiro[4,5]decane (prepared using the process described in Preparation 2 above using the appropriate starting material from Preparation 1(a) above), and 4.2 g of anhydrous potassium carbonate in 68 ml of anhydrous acetone at room temperature over 1 hour while stirring, then the reaction mixture is stirred at room temperature for an additional 1 hour. After filtering off the inorganic salts and evaporating the solvent under reduced pressure, the residue is taken up in benzene, washed with water, dried over anhydrous sodium sulfate and then the solution is evaporated to a tenth of its volume under reduced pressure. The product is precipitated by adding n-hexane to the evaporation residue. The crystals are filtered off and dried to give the title compound in 86% yield, m.p.: 128-129 °C.
Analysis:

| Calculated for $C_{26}H_{30}F_2N_2O_3$ | | | | |
|---|---|---|---|---|
| | C 68.40; | H 6.62; | F 8.32; | N 6.14%; |
| found: | C 68.44; | H 6.79; | F 8.50; | N 6.12%. |

The hydrochloride salt is precipitated by adding ethereal hydrogen chloride solution to an ethereal solution of the base, m.p.: 233-235 °C.

The following compounds may be analogously prepared using the process as described in the preceding Example and by using the appropriate starting materials.

8-(3,3-diphenyl-2-propenyl)-4-hydroxy-3-isopropyl-4-methyl-2-oxo-1-oxa-3,8-diazaspiro[4,5]decane hydrochloride, m.p.: 219-221 °C;

8-[3,3-bis(4-fluorophenyl)-2-propenyl]-4-hydroxy-3-isopropyl-4-methyl-2-oxo-1-oxa-3,8-diazaspiro[4,5]-decane hydrochloride, m.p.: 255-260 °C.

Example 2

3-n-butyl-8-(3,3-diphenyl-2-propenyl)-4-hydroxy-4-methyl-2-oxo-1-oxa-3,8-diazaspiro[4,5]decane hydrochloride

(a) 10.7 g of 4,4-bis(4-chlorophenyl)-3-butenyl bromide are added to the solution of 4.5 g of 3-butyl-4-methylene-2-oxo-1-oxa-3,8-diazaspiro[4,5]decane in 45 ml of acetone containing 4.20 g of anhydrous potassium carbonate and 0.5 g of potassium iodide. The heterogeneous reaction mixture is refluxed under nitrogen while stirring for 5 hours. After evaporating the solvent under reduced pressure, water is added to the residue and extracted with benzene. The benzene phase is washed with water until halide-free, filtered through a silica gel bed and evaporated under reduced pressure. The residue is recrystallized from ethanol to give the product in 79.0% yield, m.p.: 110-111 °C.
Analysis:

| Calculated for $C_{28}H_{32}Cl_2N_2O_2$ | | | | |
|---|---|---|---|---|
| | C 67.33; | H 6.46; | Cl 14.20; | N 5.61%; |
| found: | C 67.50; | H 6.44; | Cl 14.25; | N 5.80%. |

Using appropriate starting materials, the following compounds may be prepared analogously using the process described in the preceding Preparation:

8-[4,4-bis(4-fluorophenyl)-3-butenyl]-3-methyl-4-methylene-2-oxo-1-oxa-3,8-diazaspiro[4,5]decane, m.p.: 127.5-128.5

3-butyl-8-(3,3-diphenyl-2-propenyl)-4-methylene-2-oxo-1-oxa-3,8-diazaspiro[4,5]decane, m.p.: 138-139 °C;

(b) 92 ml of a hydrochloric acid solution of 3 mol/litre concentration are dropped into a solution of 5.2 g of 3-n-butyl-8-(3,3-diphenyl-2-propenyl)-4-methylene-2-oxo-1-oxa-3,8-diazaspiro[4,5]decane in 10.4 ml of 99% formic acid under stirring. Thereafter, the reaction mixture is stirred at 0 to 5 °C for 30 minutes. After filtering off, the crystalline precipitate is washed with water and dried to obtain the title product in 97% yield, m.p.: 104-106 °C.

Analysis of the base:

| Calculated for $C_{27}H_{34}N_2O_3$ | | | |
|---|---|---|---|
| | C 74.62; | H 7.89; | N 6.45%; |
| found: | C 74.55; | H 8.01; | N 6.56%. |

Example 3

8-[4,4-bis(4-fluorophenyl)-3-butenyl]-4-hydroxy-3-isopropyl-4-methyl-2-oxo-1-oxa-3,8-diazaspiro[4,5]decane hydrochloride

(a) A mixture containing 11.0 g of 1-[4,4-bis(4-fluorophenyl)-3-butenyl]-4-ethynyl-4-hydroxypiperidine, 6.0 g of 1-naphthyl isocyanate, 0.2 g of anhydrous potassium acetate and 36 ml of α-picoline is refluxed under nitrogen while stirring for 5 hours. After evaporating the solvent under reduced pressure, the residue is taken up in benzene, filtered through an aluminum oxide column and then evaporated under reduced pressure. The residue is recrystallized from methanol under clarifying by activated carbon to give the title product in 76.5% yield, m.p.: 99-101 °C.

Analysis:

| Calculated for $C_{34}H_{30}F_2N_2O_2$ | | | | |
|---|---|---|---|---|
| | C 76.10; | H 5.63; | F 7.08; | N 5.22%; |
| found: | C 76.23; | H 5.76; | F 7.13; | N 5.25%. |

By using the appropriate starting materials, the following compound may be prepared analogously using the process described in the preceding Preparation:

8-[4,4-bis(4-fluorophenyl)-3-butenyl]-3-isopropyl-4-methylene-2-oxo-1-oxa-3,8-diazaspiro[4,5]decane, m.p.: 135-136 °C.

(b) 11.3 g of 8-[4,4-bis(4-fluorophenyl)-3-butenyl]-3-isopropyl-4-methylene-2-oxo-1-oxa-3,8-diazaspiro-[4,5]decane are stirred with 100 ml of 1 mol/litre hydrochloric acid at room temperature for 18 hours, then the crystalline precipitate is filtered off, washed with water, cooled to 5 °C and dried under reduced pressure to give the title hydrochloride in 96% yield, m.p.: 252-255 °C (with decomposition).

The base is liberated from the hydrochloride by adding saturated aqueous sodium hydrogen carbonate solution, and extracted into chloroform. The organic layer is washed with water, dried over anhydrous magnesium sulfate and the solvent is distilled off under reduced pressure. After recrystallizing the residue from benzene, the title base is obtained in 87% yield, m.p.: 175-176 °C.

Analysis:

| Calculated for $C_{27}H_{32}F_2N_2O_3$ | | | | |
|---|---|---|---|---|
| | C 68.91; | H 6.86; | F 8.08; | N 5.95%; |
| found: | C 69.12; | H 6.94; | F 8.13; | N 6.10%. |

Example 4

8-(3,3-diphenyl-2-propenyl)-4-hydroxy-4-methyl-2-oxo-3-propyl-1-oxa-3,8-diazaspiro[4,5]decane hydrochloride

A solution containing 12.6 g of 4-acetyl-1-(3,3-diphenyl-2-propenyl)-4-propylcarbamoyloxypiperidine and 0.4 g of sodium methoxide in 80 ml of methanol is refluxed under argon for 4 to 5 hours, then the solvent is evaporated under reduced pressure. The residue is stirred with 50 ml of 1.0 mol/litre hydrochloric acid at 0 to 5 °C for 15 minutes. After filtering off, the crystalline precipitate is washed with ice-cold water and dried to obtain the product hydrochloride in 82.4% yield, m.p.: 132-134 °C.
Analysis of the base:

| Calculated for $C_{26}H_{32}N_2O_3$ | | | |
|---|---|---|---|
| | C 74.25; | H 7.67; | N 6.66%; |
| found: | C 74.40; | H 7.65; | N 6.53%. |

Example 5

8-[4,4-bis(4-fluorophenyl)-3-butenyl]-3-ethyl-4-hydroxy-4-methyl-2-oxo-1-oxa-3,8-diazaspiro[4,5]decane hydrochloride

(a) A mixture containing 7.8 g of 3-ethyl-4-methylene-2-oxo-1-oxa-3,8-diazaspiro[4,5]decane, 8.5 ml of anhydrous triethylamine, 80 ml of anhydrous methyl ethyl ketone, 0.3 g of potassium iodide and 16.7 g of 4,4-bis(4-fluorophenyl)-3-butenyl chloride is refluxed under nitrogen while stirring for 15 hours, then the solvent is distilled off under reduced pressure. After adding benzene and water to the residue, the benzene layer is washed with water to neutral, dried over anhydrous magnesium sulfate, filtered through an aluminum oxide bed and evaporated under reduced pressure. After recrystallizing the residue from ethanol, the title product is obtained in 68.7% yield, m.p.: 138-139 °C.
Analysis:

| Calculated for $C_{26}H_{28}F_2N_2O_2$ | | | | |
|---|---|---|---|---|
| | C 71.21; | H 6.44; | F 8.66; | N 6.39%; |
| found: | C 71.32; | H 6.46; | F 8.75; | N 6.58%. |

(b) 7.0 g of 8-[4,4-bis(4-fluorophenyl)-3-butenyl]-3-ethyl-4-methylene-2-oxo-1-oxa-3,8-diazaspiro[4,5]-decane are boiled with 320 ml of 0.1 mol/litre hydrochloric acid while stirring for 1 hour . After cooling down the reaction mixture to 5 °C and filtering it, the crystalline precipitate is washed with water and dried to obtain the title hydrochloride in 92.0% yield, m.p.: 238-240 °C (with decomposition).

The base is liberated from the hydrochloride by adding aqueous sodium hydroxide solution.
Analysis of the base:

| Calculated for $C_{26}H_{30}F_2N_2O_3$ | | | | |
|---|---|---|---|---|
| | C 68.40; | H 6.62; | F 8.32; | N 6.14%; |
| found: | C 68.36; | H 6.68; | F 8.50; | N 6.25%. |

Example 6

8-[4,4-bis(4-fluorophenyl)-3-butenyl]-3-decyl-4-hydroxy-4-methyl-2-oxo-1-oxa-3,8-diazaspiro[4,5]decane hydrochloride

5.5 ml of decylamine are added portionwise to a solution of 10.3 g of 8-[4,4-bis(4-fluorophenyl)-3-butenyl]-4-methylene-2-oxo-1,3-dioxa-8-azaspiro[4,5]decane (prepared using the process as described in

Preparation 3 above using the appropriate azaspiro[4,5] decane as prepared in a manner analogous to the process described in Preparation 1), in 20 ml of benzene while stirring. The reaction is mildly exothermic and the temperature of the reaction mixture increases to 44-45 °C. After addition, the reaction mixture is stirred at room temperature for 18 to 20 hours. The crystalline precipitate is filtered off, washed with hexane and dried. The base is transformed into its hydrochloride salt by adding ethereal hydrogen chloride solution to obtain the title hydrochloride in 78.6% yield, m.p.: 140-141 °C.

Analysis of the base:

| Calculated for $C_{34}H_{46}F_2N_2O_3$ | | | | |
|---|---|---|---|---|
| | C 71.80; | H 8.15; | F 6.68; | N 4.93%; |
| found: | C 71.88; | H 8.33; | F 6.61; | N 5.11%. |

The following compounds may be analogously prepared using the process as described in the preceding Example and by using the appropriate starting materials.

8-[4,4-bis(4-fluorophenyl)-3-butenyl]-3-[2-(3,4-dimethoxyphenyl)ethyl]-4-hydroxy-4-methyl-2-oxo-1-oxa-3,8-diazaspiro[4,5]decane, m.p: 143-144 °C; the hydrogen maleate salt decomposes at 129-132 °C;

8-[4,4-bis(4-fluorophenyl)-3-butenyl]-4-hydroxy-4-methyl-2-oxo-3-propyl-1-oxa-3,8-diazaspiro[4,5]decane hydrochloride, m.p: 247-250 °C (with decomposition);

8-[4,4-bis(4-fluorophenyl)-3-butenyl]-3-cyclohexyl-4-hydroxy-4-methyl-2-oxo-1-oxa-3,8-diazaspiro[4,5]-decane hydrochloride, m.p: 256-258 °C (with decomposition);

8-[3,3-bis(4-fluorophenyl)-2-propenyl]-3-butyl-4-hydroxy-4-methyl-2-oxo-1-oxa-3,8-diazaspiro[4,5]decane hydrochloride, m.p: 228-230 °C (with decomposition);

3-benzyl-8-[4,4-bis(4-fluorophenyl)-3-butenyl]-4-hydroxy-4-methyl-2-oxo-1-oxa-3,8-diazaspiro[4,5]decane hydrochloride, m.p: 179-181 °C (with decomposition);

8-[4,4-bis(4-fluorophenyl)-3-butenyl]-4-hydroxy-4-methyl-2-oxo-1-oxa-3,8-diazaspiro[4,5]decane hydrochloride, m.p: 155-157 °C (with decomposition);

8-[4,4-bis(4-fluorophenyl)-3-butenyl]-4-hydroxy-4-methyl-3-(1-naphthyl)-2-oxo-1-oxa-3,8-diazaspiro[4,5]-decane hydrochloride, m.p: 230-233 °C (with decomposition);

3,4-dimethyl-8-(3,3-diphenyl-2-propenyl)-4-hydroxy-2-oxo-1-oxa-3,8-diazaspiro[4,5]decane hydrochloride, m.p: 211-213 °C (with decomposition);

8-[4,4-bis(4-fluorophenyl)-3-butenyl]-3-tert-butyl-4-hydroxy-4-methyl-2-oxo-1-oxa-3,8-diazaspiro[4,5]-decane hydrochloride, m.p: 220-224 °C (with decomposition);

8-[3,3-bis(4-fluorophenyl)-2-propenyl]-3,4-dimethyl-4-hydroxy-2-oxo-1-oxa-3,8-diazaspiro[4,5]decane hydrochloride, m.p: 238-240 °C (with decomposition);

8-[4,4-bis(4-fluorophenyl)-3-butenyl]-4-hydroxy-4-methyl-2-oxo-3-phenyl-1-oxa-3,8-diazaspiro[4,5]decane hydrochloride, m.p: 179-181 °C (with decomposition);

8-[4,4-bis(4-fluorophenyl)-3-butenyl]-3,4-dimethyl-4-hydroxy-2-oxo-1-oxa-3,8-diazaspiro[4,5]decane, m.p: 144-145 °C; the hydrochloride decomposes at 203-204 °C;

8-[4,4-bis(4-fluorophenyl)-3-butenyl]-3-butyl-4-hydroxy-4-methyl-2-oxo-1-oxa-3,8-diazaspiro[4,5]decane hydrochloride, m.p: 229-231 °C (with decomposition).

## Example 7

### 3-(4-chlorophenyl)-8-(3,3-diphenyl-2-propenyl)-4-hydroxy-4-methyl-2-oxo-1-oxa-3,8-diazaspiro[4,5]decane hydrochloride

A solution containing 6.4 g of 4-acetyl-1-(3,3-diphenyl-2-propenyl)-4-hydroxypiperidine and 3.4 g of 4-chlorophenyl isocyanate in 30 ml of tripropylamine is gently refluxed under argon while stirring for 3 hours, then the solvent is distilled off under reduced pressure. After adding benzene to the evaporation residue, the insoluble materials are filtered off, the benzene solution is filtered through a silica gel layer and the solution is evaporated under reduced pressure. After recrystallizing the crude product from a mixture of ethanol and hexane under clarifying with activated carbon, the resultant base is converted into its hydrochloride salt by adding a solution of hydrogen chloride in diisopropyl ether. The title hydrochloride is obtained in 47.3% yield, m.p.: 227-230 °C (with decomposition).

Analysis of the base:

| Calculated for $C_{29}H_{29}ClN_2O_3$ | | | | |
|---|---|---|---|---|
| | C 71.22; | H 5.98; | Cl 7.25; | N 5.73%; |
| found: | C 71.29; | H 6.14; | Cl 7.40; | N 5.60%. |

Example 8

8-[4,4-bis(4-chlorophenyl)-3-butenyl]-3-butyl-4-hydroxy-4-methyl-2-oxo-1-oxa-3,8-diazaspiro[4,5]decane hydrochloride

A mixture containing 7.27 g of 3-butyl-4-hydroxy-4-methyl-2-oxo-1-oxa-3,8-diazaspiro[4,5]decane (prepared using the process in Preparation 2 above using the appropriate starting material from Preparation 1(a) above,) 12.89 g of 4,4-bis(4-chlorophenyl)-3-butenyl bromide, 4.98 g of anhydrous potassium carbonate and 0.6 g of potassium iodide in 73 ml of methyl isobutyl ketone is gently refluxed under argon while stirring for 6 hours. After cooling down, the inorganic salts are filtered off, washed with methyl isobutyl ketone, the filtrate is washed with water to neutral, dried over anhydrous magnesium sulfate and then the solvent is distilled off under reduced pressure. After recrystallizing the evaporation residue from ethanol, the product obtained is dissolved in ether and the hydrochloride is precipitated by adding ethereal hydrogen chloride solution to obtain 79.3% of the title hydrochloride, m.p.: 230-233 °C (with decomposition).
Analysis of the base:

| Calculated for $C_{28}H_{34}Cl_2N_2O_3$ | | | | |
|---|---|---|---|---|
| | C 64.89; | H 6.62; | Cl 13.70; | N 5.41%; |
| found: | C 65.04; | H 6.66; | Cl 13.62; | N 5.48%. |

Example 9

8-[3,3-bis(4-fluorophenyl)-2-propenyl]-8-methyl-4-hydroxy-4-methyl-2-oxo-3-propyl-1-oxa-3,8-diazaspiro[4,5]-decan-8-ium iodide

A mixture of 4.1 g of 8-[3,3-bis(4-fluorophenyl)-2-propenyl]-4-hydroxy-4-methyl-2-oxo-3-propyl-1-oxa-3,8-diazaspiro[4,5]decane [Example 1 above] and 0.7 ml of methyl iodide in 400 ml of methyl isobutyl ketone is refluxed for 2 hours. After cooling down, the crystalline precipitate is filtered off, washed with diisopropyl ether, cooled to 0 °C and dried to give 91.3% yield of the title quaternary ammonium salt, m.p.: 232-233 °C.

Examples of pharmaceutical compounds that may be prepared using compounds according to the present invention include:

a) Preparation of tablets

50.0 g of active ingredient are mixed together with 92 g of lactose, 40 g of potato starch, 4 g of polyvinylpyrrolidone, 6 g of talc, 1 g of magnesium stearate, 1 g of colloidal silicon dioxide (Aerosil) and 6 g of ultraamylopectin and, after wet granulatlon, the product obtained is compressed into tablets containing 50 mg of the active ingredient each.

b) Preparation of dragées

After coating the tablets prepared as described above in a known manner with a layer comprising sugar and talc, the dragées obtained are polished with a mixture of bees wax and carnauba wax to obtain dragées weighing 250 mg each.

c) Preparation of capsules

100 g of active ingredient are thoroughly mixed together with 30 g of sodium lauryl sulfate, 280 g of starch. 280 g of lactose, 4 g of colloidal silicon dioxide (Aerosil) and 6 g of magnesium stearate, then the mixture is sieved and filled into hard gelatine capsules to obtain capsules containing 100 mg of active ingredient each.

d) Preparation of suppositories

(Note: all amounts are calculated for one suppository)

100.0 mg of active ingredient are thoroughly mixed together with 200.0 mg of lactose. 1700.0 mg of suppository base (e.g. Witepsol H) are made molten, cooled to 35 °C and the mixture of the active ingredient and lactose is mixed thereinto using a homogenizer. The product obtained is poured into cooled conic moulds. Each suppository weighes 2000 mg.

e) Preparation of a suspension

| Components in 100 ml of the suspension: | |
|---|---|
| Active ingredient | 1.00 g |
| Sodium hydroxide | 0.26 g |
| Citric acid | 0.30 g |
| Nipagin (methyl 4-hydroxybenzoate sodium salt) | 0.10 g |
| Carbopol 940 (polyacrylic acid) | 0.30 g |
| 96% Ethanol | 1.00 g |
| Raspberry flavour | 0.60 g |
| Sorbitol (Aqueous solution of 70%) | 71.00 g |
| Distilled water up to | 100.00 ml |

"Aerosil", "Witepsol", "Nipagin" and "Carbopol" are all Registered Trade Marks.

After adding Carbapol in little portions to the solution of Nipagin and citric acid in 20 ml of distilled water under vigorous stirring, the solution obtained is left to stand for 10 to 12 hours. Subsequently, the amount given above of sodium hydroxyde dissolved in 1 ml of distilled water, the aqueous solution of sorbitol and finally the ethanolic solution of the rapsberry flavour are dropped in under stirring. The active ingredient is added in small portions to this mixture and suspended by using a submerged homogenizer. Finally, the suspension is supplemented to 100 ml by adding sufficient distilled water and the syrupy suspension is led through a colloid mill.

## Claims

**Claims for the following Contracting States : AT, BE, CH, DE, DK, FR, GB, IT, LI, LU, NL, SE**

**1.** A compound of formula (I),

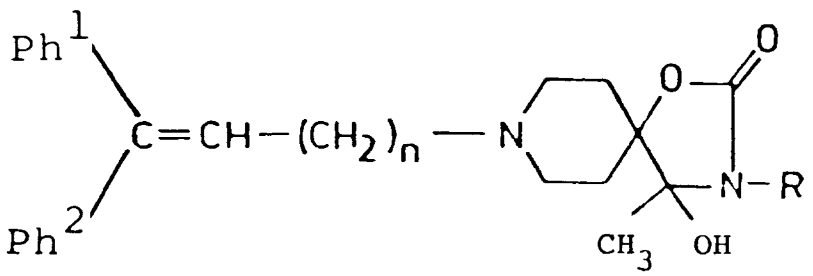

(I)

wherein

R represents a hydrogen atom or a $C_{1-12}$alkyl or $C_{3-6}$cycloalkyl group, or a carbocyclic $C_{6-10}$aryl or carbocyclic $C_{6-10}$aryl-$C_{1-4}$alkyl group optionally substituted

on the aromatic ring by one or more halogen atoms, $C_{1-4}$alkyl or $C_{1-4}$alkoxy groups;

Ph[1] and Ph[2], which may be the same or different, each represent a phenyl group unsubstituted or substitued by one or more halogen atoms or $C_{1-4}$alkyl, $C_{1-4}$alkoxy, trihalomethyl groups or hydroxyl groups optionally esterified by a $C_{1-4}$alkanoic acid; and

n is 1 or 2,

and optical isomers and mixtures thereof, and all solvates, hydrates, acid addition and quaternary ammonium salts thereof.

**2.** A compound as defined in claim 1 selected from the group comprising:

8-[4,4-bis(4-fluorophenyl)-3-butenyl]-4-hydroxy-4-methyl 2-oxo-1-oxa-3,8-diazaspiro[4,5]decane hydrochloride,

8-[4,4-bis(4-fluorophenyl)-3-butenyl]-3,4-dimethyl-4-hydroxy-2-oxo-1-oxa-3,8-diazaspiro[4,5]decane hydrochloride,

8-[4,4-bis(4-fluorophenyl)-3-butenyl]-3-tert-butyl-4-hydroxy-4-methyl-2-oxo-1-oxa-3,8-diazaspiro[4,5]-decane hydrochloride,

8-[4,4-bis(4-fluorophenyl)-3-butenyl]-3-butyl-4-hydroxy-4-methyl-2-oxo-1-oxa-3,8-diazaspiro[4,5]-decane hydrochloride,

3-benzyl-8-[4,4-bis(4-fluorophenyl)-3-butenyl]-4-hydroxy-4-methyl-2-oxo-1-oxa-3,8-diazapiro[4,5]-decane hydrochloride,

and optical isomers and mixtures thereof and all solvates, hydrates, acid addition and quaternary ammonium salts thereof.

**3.** A pharmaceutical composition which comprises as active ingredient a compound of formula (I) as defined in claim 1 or a pharmaceutically acceptable acid addition or quaternary ammonium salt thereof in admixture with a pharmaceutically acceptable carrier, diluent or excipient.

**4.** A process for the preparation of 2-oxo-3,8-diazaspiro[4,5]decane derivatives of formula (I) as defined in claim 1 which comprises

a) reacting a compound of formula (II)

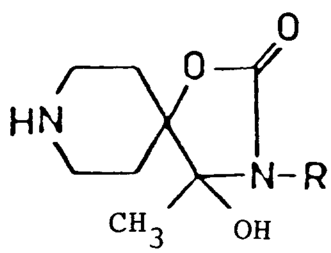

(II)

wherein R is as defined in claim 1, with a compound of formula (III),

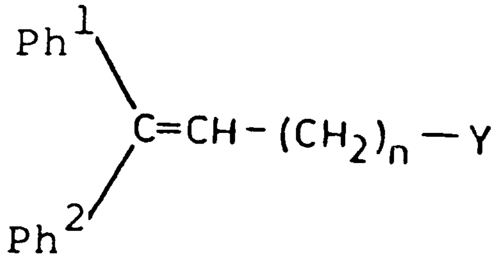

(III)

wherein Ph[1], Ph[2] and n are as defined in claim 1 and Y represents a halogen or a $C_{1-4}$alkylsulfonyloxy or arylsulfonyloxy group;

or

b) reacting a compound of formula (VII),

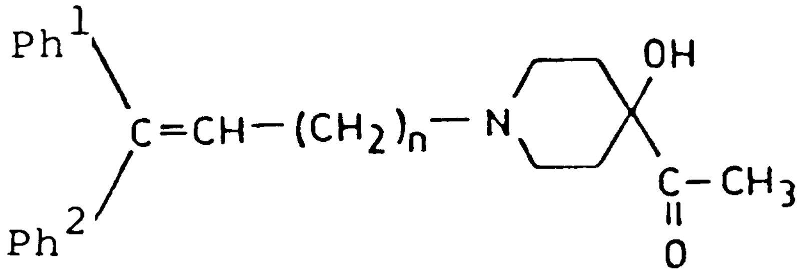

(VII)

wherein $Ph^1$, $Ph^2$ and n are as defined above , with an isocyanate of the formula R-NCO, wherein R is as defined above other than hydrogen, followed by cyclizing the resultant compound of formula (VIII),

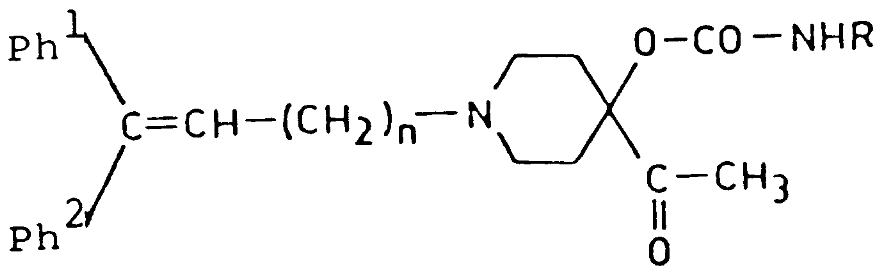

(VIII)

wherein R, $Ph^1$, $Ph^2$ and n are as defined above;

or

c) cyclizing a compound of formula (VIII) as defined above;

or

d) reacting a compound of formula (VI),

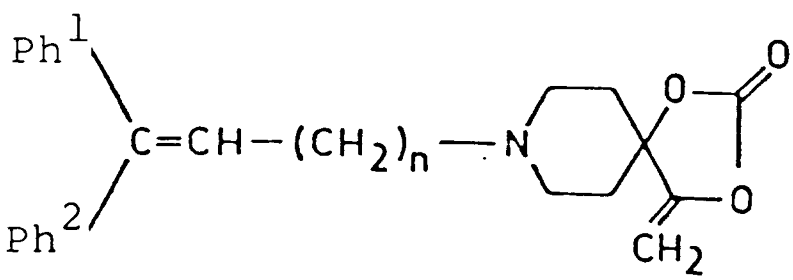

(VI)

wherein $Ph^1$, $Ph^2$ and n are as defined above, with an amine of formula $R\text{-}NH_2$, wherein R is as defined above;

or

e) hydrating a compound of formula (IV),

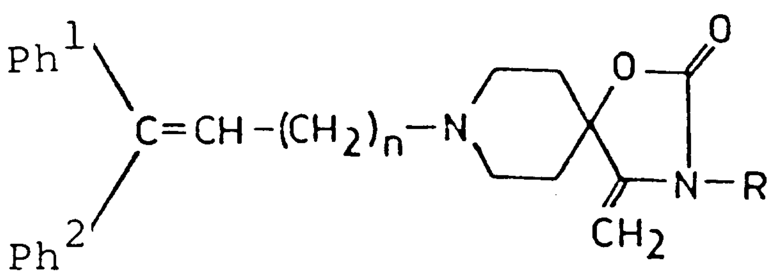

(IV)

wherein R, $Ph^1$, $Ph^2$ and n are as defined above, in an acidic medium;
followed if desired or necessary,
by transforming a functional group of a thus-obtained compound of formula (I) as defined above into another compound of formula (I) as defined in claim 1 in a known manner;
and/or
reacting a thus-obtained compound of the formula (I) as defined above with an acid to give an acid addition salt and/or treating a compound of formula (I) as defined above, obtained as a salt, with a base to liberate the free basic form thereof and/or converting a thus-obtained compound of formula (I) as defined above into its quaternary ammonium salt.

**5.** A process as claimed in claim 4 process a) wherein Y is a chlorine or bromine atom.

**6.** A process as claimed in claim 4 process a) wherein Y is a methylsulfonyloxy or p-toluenesulfonyloxy group.

**7.** A process as claimed in claim 4 process a) or claim 5 which comprises carrying out the reaction in the presence of potassium iodide in a ketone-type solvent.

**8.** A process as claimed in claim 4 process b) which comprises carrying out all the reaction steps in a triisopropylamine medium and at the boiling point of the reaction mixture.

**9.** A process for the preparation of a pharmaceutical composition as defined in claim 3 which comprises admixing a compound of formula (I) as defined in claim 1 or a pharmaceutically acceptable acid addition or quaternary ammonium salt thereof with a pharmaceutically acceptable carrier, diluent or excipient.

**10.** The use of a compound of formula (I) as defined in claim 1 in the manufacture of a medicament for the treatment of disturbances of the cognitive functions of mammals.

**Claims for the following Contracting States : ES, GR**

**1.** A process for the preparation of a compound of formula (I),

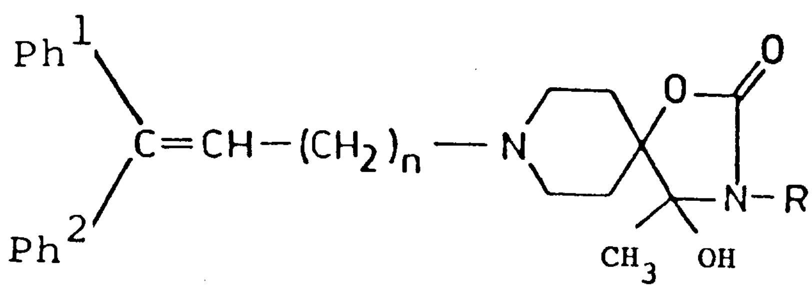 (I)

wherein

| | |
|---|---|
| R | represents a hydrogen atom or a $C_{1-12}$alkyl or $C_{3-6}$cycloalkyl group, or a carbocyclic $C_{6-10}$aryl or carbocyclic $C_{6-10}$aryl-$C_{1-4}$alkyl group optionally substituted on the aromatic ring by one or more halogen atoms, $C_{1-4}$alkyl or $C_{1-4}$alkoxy groups; |
| $Ph^1$ and $Ph^2$, | which may be the same or different, each represent a phenyl group unsubstituted or substitued by one or more halogen atoms or $C_{1-4}$alkyl, $C_{1-4}$alkoxy, trihalomethyl groups or hydroxyl groups optionally esterified by a $C_{1-4}$alkanoic acid; and |
| n | is 1 or 2, |

and optical isomers and mixtures thereof, and all solvates, hydrates, acid addition and quaternary ammonium salts thereof which comprises

a) reacting a compound of formula (II)

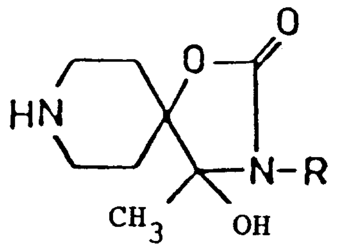

(II)

wherein R is as defined above with a compound of formula (III),

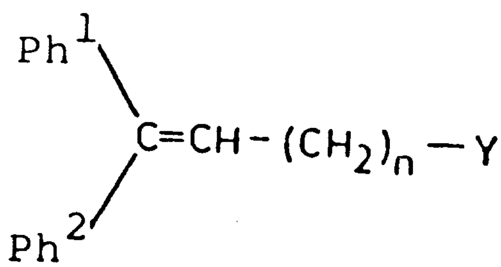

(III)

wherein $Ph^1$, $Ph^2$ and n are as defined above and Y represents a halogen or a $C_{1-4}$alkylsulfonyloxy or arylsulfonyloxy group;

or

b) reacting a compound of formula (VII),

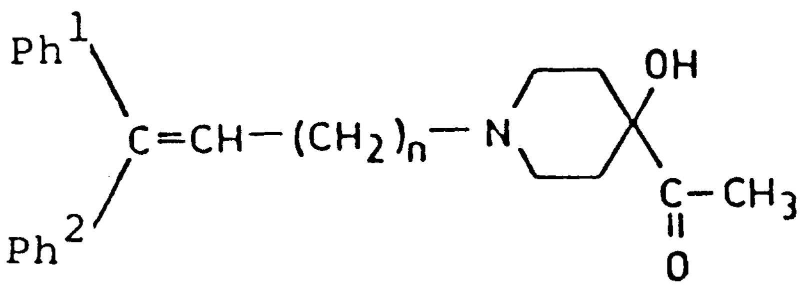

(VII)

wherein $Ph^1$, $Ph^2$ and n are as defined above , with an isocyanate of the formula R-NCO, wherein R is as defined above other than hydrogen, followed by cyclizing the resultant compound of formula (VIII),

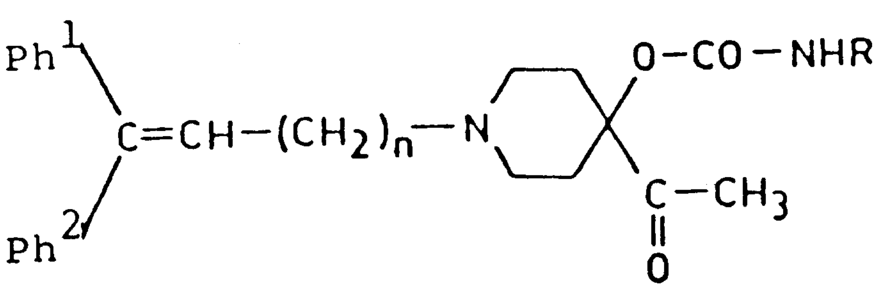

(VIII)

wherein R, $Ph^1$, $Ph^2$ and n are as defined above;

or

c) cyclizing a compound of formula (VIII) as defined above;
or
d) reacting a compound of formula (VI),

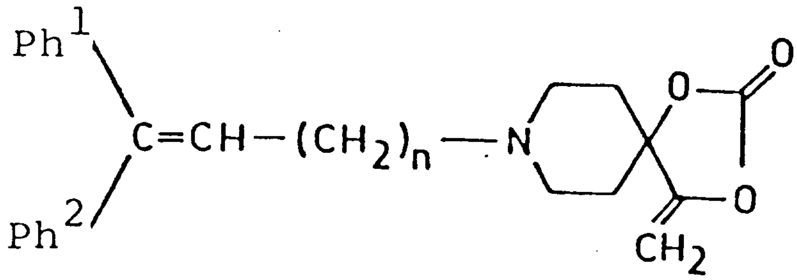

(VI)

wherein $Ph^1$, $Ph^2$ and n are as defined above, with an amine of formula $R\text{-}NH_2$, wherein R is as defined above;
or
e) hydrating a compound of formula (IV),

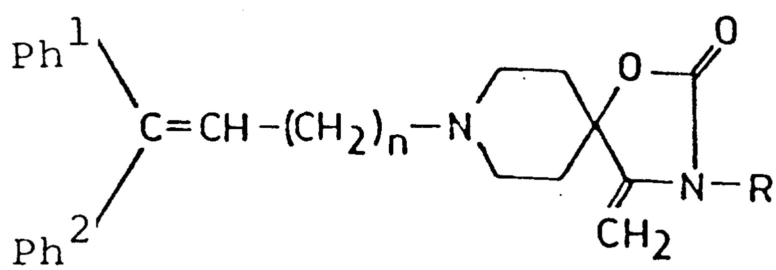

(IV)

wherein R, $Ph^1$, $Ph^2$ and n are as defined above, in an acidic medium;
followed if desired or necessary,
by transforming a functional group of a thus-obtained compound of formula (I) as defined above into another compound of formula (I) as defined above in a known manner;
and/or
reacting a thus-obtained compound of the formula (I) as defined above with an acid to give an acid addition salt and/or treating a compound of formula (I) as defined above, obtained as a salt, with a base to liberate the free basic form thereof and/or converting a thus-obtained compound of formula (I) as defined above into its quaternary ammonium salt.

**2.** A process as claimed in claim 1 process a) wherein Y is a chlorine or bromine atom.

**3.** A process as claimed in claim 1 process a) wherein Y is a methylsulfonyloxy or p-toluenesulfonyloxy group.

**4.** A process as claimed in claim 1 process a) or claim 2 which comprises carrying out the reaction in the presence of potassium iodide in a ketone-type solvent.

**5.** A process as claimed in claim 1 process b) which comprises carrying out all the reaction steps in a triisopropylamine medium and at the boiling point of the reaction mixture.

**6.** A process as defined in claim 1 for the preparation of a compound of formula (I) as defined in claim 1 selected from the group comprising:

8-[4,4-bis(4-fluorophenyl)-3-butenyl]-4-hydroxy-4-methyl 2-oxo-1-oxa-3,8-diazaspiro[4,5]decane hydrochloride,

8-[4,4-bis(4-fluorophenyl)-3-butenyl]-3,4-dimethyl-4-hydroxy-2-oxo-1-oxa-3,8-diazaspiro[4,5]decane

hydrochloride,

8-[4,4-bis(4-fluorophenyl)-3-butenyl]-3-tert-butyl-4-hydroxy-4-methyl-2-oxo-1-oxa-3,8-diazaspiro[4,5]-decane hydrochloride,

8-[4,4-bis(4-fluorophenyl)-3-butenyl]-3-butyl-4-hydroxy-4-methyl-2-oxo-1-oxa-3,8-diazaspiro[4,5]-decane hydrochloride,

3-benzyl-8-[4,4-bis(4-fluorophenyl)-3-butenyl]-4-hydroxy-4-methyl-2-oxo-1-oxa-3,8-diazapiro[4,5]-decane hydrochloride,

and optical isomers and mixtures thereof and all solvates, hydrates, acid addition and quaternary ammonium salts thereof.

**7.** A process for the preparation of a pharmaceutical composition which comprises as active ingredient a compound of formula (I) as defined in claim 1 or a pharmaceutically acceptable acid addition or quaternary ammonium salt thereof, wherein the active ingredient is admixed with a pharmaceutically acceptable carrier, diluent or excipient.

**8.** The use of a compound of formula (I) as defined in claim 1 in the manufacture of a medicament for the treatment of disturbances of the cognitive functions of mammals.

**Patentansprüche**

**Patentansprüche für folgende Vertragsstaaten : AT, BE, CH, DE, DK, FR, GB, IT, LI, LU, NL, SE**

**1.** Verbindung der Formel (I),

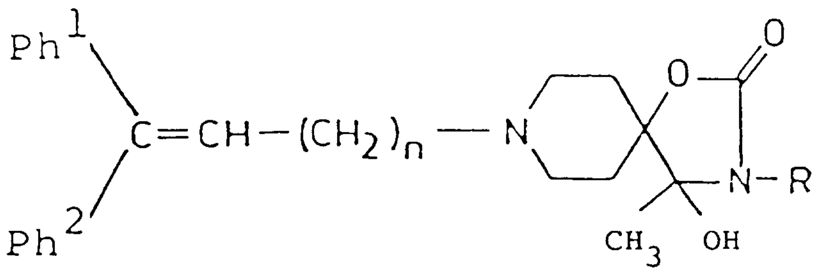

(I)

worin

R ein Wasserstoffatom oder eine $C_{1-12}$-Alkyl- oder $C_{3-6}$-Cycloalkylgruppe oder eine carbocyclische $C_{6-10}$-Aryl- oder carbocyclische $C_{6-10}$-Aryl-$C_{1-4}$-alkylgruppe darstellt, die gegebenenfalls am aromatischen Ring mit einem oder mehreren Halogenatomen, $C_{1-4}$-Alkyl- oder $C_{1-4}$-Alkoxygruppen substituiert sind;

$Ph^1$ und $Ph^2$, die gleich oder verschieden sein können, jeweils eine Phenylgruppe darstellen, die unsubstituiert oder mit einem oder mehreren Halogenatomen oder $C_{1-4}$-Alkyl-, $C_{1-4}$-Alkoxy-, Trihalomethylgruppen oder Hydroxylgruppen, die gegebenenfalls mit einer $C_{1-4}$-Alkansäure verestert sind, substituiert ist; und

n 1 oder 2 ist,

und deren optische Isomere und Mischungen und alle ihre Solvate, Hydrate, Säureadditions- und quaternären Ammoniumsalze.

**2.** Verbindung gemäß Anspruch 1, die aus der Gruppe ausgewählt ist, welche umfaßt:

8-[4,4-Bis(4-fluorphenyl)-3-butenyl]-4-hydroxy-4-methyl-2-oxo-1-oxa-3,8-diazaspiro[4,5]decan-hydrochlorid,

8-[4,4 -Bis(4-fluorphenyl)-3-butenyl]-3,4-dimethyl-4-hydroxy-2-oxo-1-oxa-3,8-diazaspiro[4,5]decan-hydrochlorid,

8-[4,4-Bis(4-fluorphenyl)-3-butenyl]-3-tert-butyl-4-hydroxy-4-methyl-2-oyo-1-oxa-3,8-diazaspiro[4,5]-decan-hydrochlorid,

8-[4,4-Bis(4-fluorphenyl)-3-butenyl]-3-butyl-4-hydroxy-4-methyl-2-oxo-1-oxa-3,8-diazaspiro[4,5]decan-hydrochlorid,

3-Benzyl-8-[4,4-bis(4-fluorphenyl)-3-butenyl]-4-hydroxy-4-methyl-2-oxo-1-oxa-3,8-diazaspiro[4,5]decan-hydrochlorid,

und ihre optischen Isomere und Mischungen und alle ihre Solvate, Hydrate, Säureadditions- und quaternären Ammoniumsalze.

3. Pharmazeutische Zusammensetzung, umfassend als Wirkstoff eine Verbindung der Formel (I) gemäß Anspruch 1 oder eines ihrer pharmazeutisch annehmbaren Säureadditions- oder quaternären Ammoniumsalze in Mischung mit einem pharmazeutisch annehmbaren Träger, Verdünnungsmittel oder Arzneiträger.

4. Verfahren zur Herstellung von 2-Oxo-3,8-diazaspiro[4,5]decan-Derivaten der Formel (I) gemäß Anspruch 1, welches umfaßt:
(a) Umsetzung einer Verbindung der Formel (II)

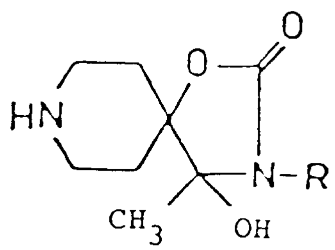

(II)

worin R wie in Anspruch 1 definiert ist, mit einer Verbindung der Formel (III),

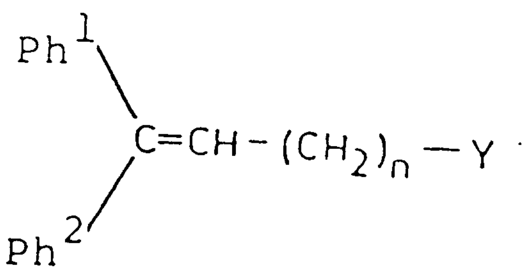

(III)

worin $Ph^1$, $Ph^2$ und n wie in Anspruch 1 definiert sind und Y ein Halogenatom oder eine $C_{1-4}$-Alkylsulfonyloxy- oder Arylsulfonyloxygruppe darstellt; oder
(b) Umsetzung einer Verbindung der Formel (VII)

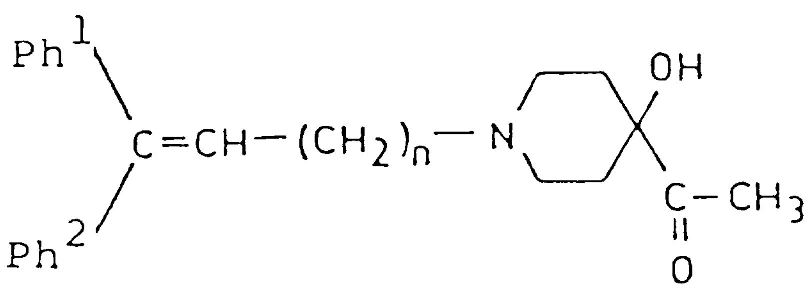

(VII)

worin $Ph^1$, $Ph^2$ und n wie oben definiert sind, mit einem Isocyanat der Formel R-NCO, worin R wie oben definiert ist außer Wasserstoff, gefolgt von Cyclisierung der resultierenden Verbindung der Formel (VIII)

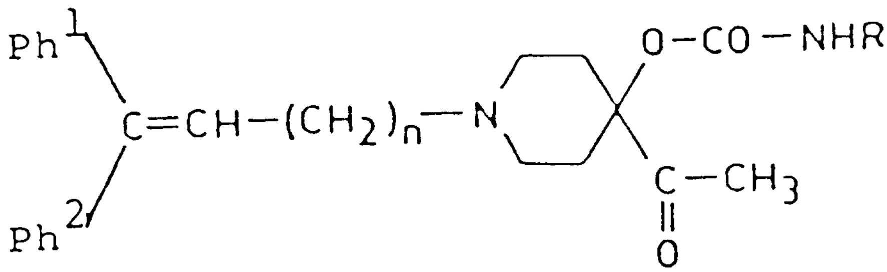

(VIII)

worin R, $Ph^1$, $Ph^2$ und n wie oben definiert sind; oder
(c) Cyclisierung einer Verbindung der wie oben definierten Formel (VIII); oder
(d) Umsetzung einer Verbindung der Formel (VI)

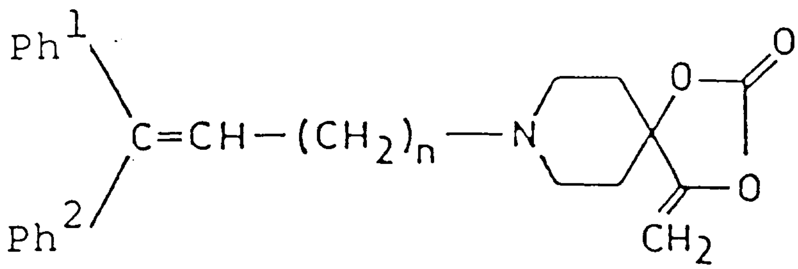

(VI)

worin $Ph^1$, $Ph^2$ und n wie oben definiert sind, mit einem Amin der Formel $R-NH_2$, worin R wie oben definiert ist; oder
(e) Hydratisierung einer Verbindung der Formel (IV)

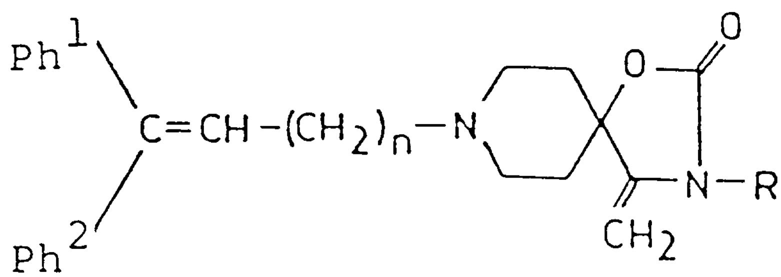

(IV)

worin R, $Ph^1$, $Ph^2$ und n wie oben definiert sind, in einem sauren Medium, gefolgt, falls gewünscht oder erforderlich, von
Umwandlung einer funktionellen Gruppe einer so erhaltenen Verbindung der wie oben definierten Formel (I) in eine andere Verbindung der wie in Anspruch 1 definierten Formel (I) auf bekannte Weise;
und/oder
Umsetzung einer so erhaltenen Verbindung der wie oben definierten Formel (I) mit einer Säure zur Bildung eines Säureadditionssalzes und/oder Behandlung einer als Salz erhaltenen Verbindung der wie oben definierten Formel (I) mit einer Base zur Freisetzung ihrer freien Base-Form und/oder Umwandlung einer so erhaltenen Verbindung der wie oben definierten Formel (I) in ihr quaternäres Ammoniumsalz.

**5.** Verfahren gemäß Anspruch 4, Verfahren (a), worin Y ein Chlor- oder Bromatom ist.

**6.** Verfahren gemäß Anspruch 4, Verfahren (a), worin Y eine Methylsulfonyloxy- oder p-Toluolsulfonyloxygruppe ist

**7.** Verfahren gemäß Anspruch 4, Verfahren (a), oder Anspruch 5, umfassend die Ausführung der Reaktion in Gegenwart von Kaliumjodid in einem Keton-Lösungsmittel.

**8.** Verfahren gemäß Anspruch 4, Verfahren (b), umfassend die Ausführung aller Reaktionsschritte in einem Triisopropylamin-Medium am Siedepunkt der Reaktionsmischung.

**9.** Verfahren zur Herstellung einer pharmazeutischen Zusammensetzung gemäß Anspruch 3, umfassend die Mischung einer Verbindung der Formel (I) gemäß Anspruch 1 oder eines ihrer pharmazeutisch annehmbaren Säureadditions- oder quaternären Ammoniumsalze mit einem pharmazeutisch annehmbaren Träger, Verdünnungsmittel oder Arzneiträger.

**10.** Verwendung einer Verbindung der Formel (I) gemäß Anspruch 1 bei der Herstellung eines Medikaments zur Behandlung von Störungen der Kognitivfunktionen von Säugern.

**Patentansprüche für folgende Vertragsstaaten : ES, GR**

**1.** Verfahren zur Herstellung einer Verbindung der Formel (I),

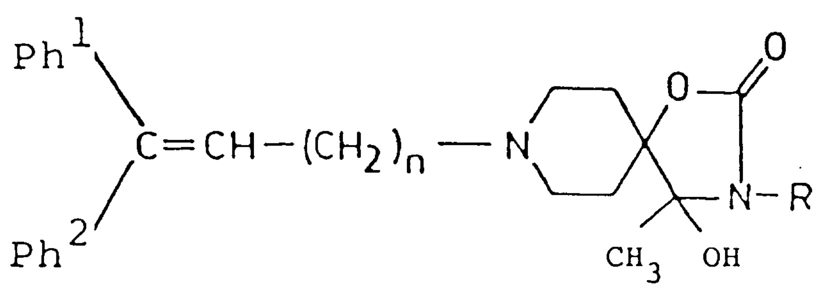

(I)

worin

| | |
|---|---|
| R | ein Wasserstoffatom oder eine $C_{1-12}$-Alkyl- oder $C_{3-6}$-Cycloalkylgruppe oder eine carbocyclische $C_{6-10}$-Aryl- oder carbocyclische $C_{6-10}$-Aryl-$C_{1-4}$-alkylgruppe darstellt, die gegebenenfalls am aromatischen Ring durch ein oder mehrere Halogenatome, $C_{1-4}$-Alkyl- oder $C_{1-4}$-Alkoxygruppen substituiert sind; |
| $Ph^1$ und $Ph^2$, | die gleich oder verschieden sein können, jeweils eine Phenylgruppe darstellen, die unsubstituiert oder mit einem oder mehreren Halogenatomen oder $C_{1-4}$-Alkyl-, $C_{1-4}$-Alkoxy-, Trihalomethylgruppen oder Hydroxylgruppen, die gegebenenfalls mit einer $C_{1-4}$-Alkansäure verestert sind, substituiert ist; und |
| n | 1 oder 2 ist, |

und deren optischer Isomere und Mischungen und allen ihren Solvaten, Hydraten, Säureadditions- und quaternären Ammoniumsalzen, welches umfaßt:

(a) Umsetzung einer Verbindung mit der Formel (II)

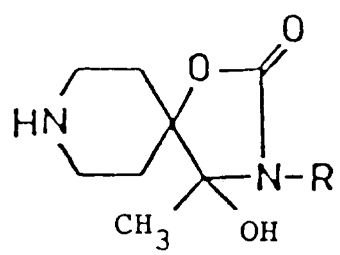

(II)

worin R wie oben definiert ist, mit einer Verbindung der Formel (III),

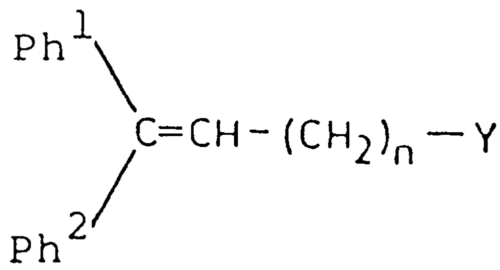

(III)

worin $Ph^1$, $Ph^2$ und n wie oben definiert sind und Y ein Halogen oder eine $C_{1-4}$-Alkylsulfonyloxy- oder Arylsulfonyloxygruppe darstellt; oder
(b) Umsetzung einer Verbindung der Formel (VII)

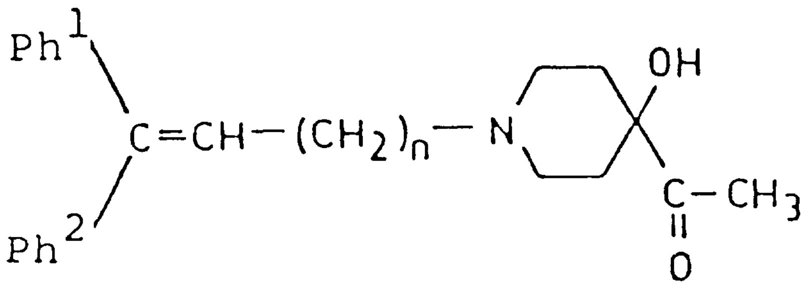

(VII)

worin $Ph^1$, $Ph^2$ und n wie oben definiert sind, mit einem Isocyanat der Formel R-NCO, worin R wie oben definiert ist außer Wasserstoff, gefolgt von Cyclisierung der resultierenden Verbindung der Formel (VIII)

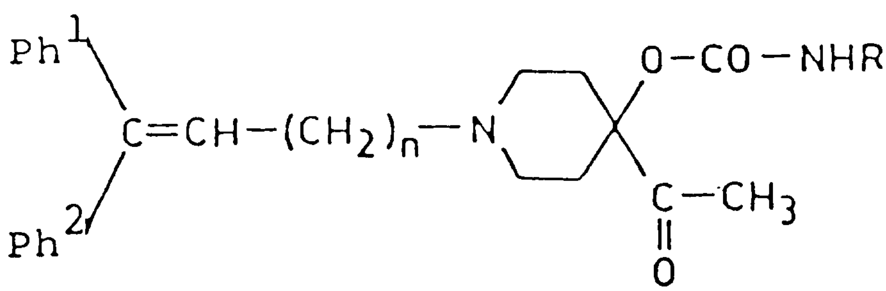

(VIII)

worin R, $Ph^1$, $Ph^2$ und n wie oben definiert sind; oder
(c) Cyclisierung einer Verbindung der wie oben definierten Formel (VIII); oder
(d) Umsetzung einer Verbindung der Formel (VI)

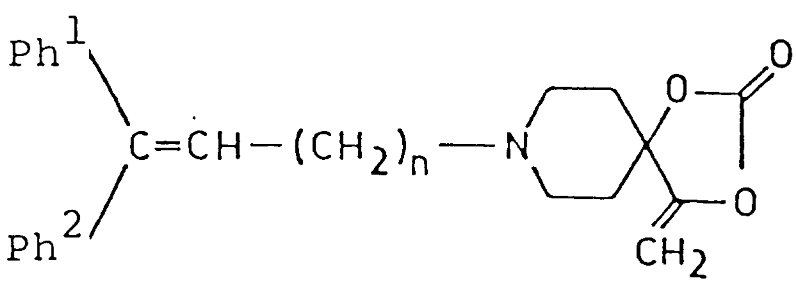

(VI)

worin $Ph^1$, $Ph^2$ und n wie oben definiert sind, mit einem Amin der Formel $R\text{-}NH_2$, worin R wie oben definiert ist; oder

(e) Hydratisierung einer Verbindung der Formel (IV)

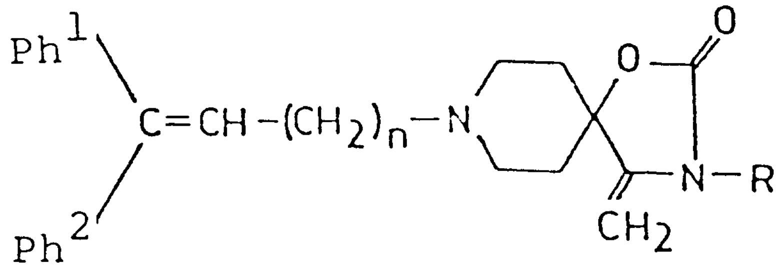

(IV)

worin R, $Ph^1$, $Ph^2$ und n wie oben definiert sind, in einem sauren Medium; gefolgt, falls gewünscht oder erforderlich, von

Umwandlung einer funktionellen Gruppe einer so erhaltenen Verbindung der wie oben definierten Formel (I) in eine andere Verbindung der wie oben definierten Formel (I) auf bekannte Weise;

und/oder

Umsetzung einer so erhaltenen Verbindung der wie oben definierten Formel (I) mit einer Säure zur Bildung eines Säureadditionssalzes und/oder Behandlung einer als Salz erhaltenen Verbindung der wie oben definierten Formel (I) mit einer Base zur Freisetzung ihrer freien Base-Form und/oder Umwandlung einer so erhaltenen Verbindung der wie oben definierten Formel (I) in ihr quaternäres Ammoniumsalz.

**2.** Verfahren gemäß Anspruch 1, Verfahren (a), worin Y ein Chlor- oder Bromatom ist.

**3.** Verfahren gemäß Anspruch 1, Verfahren (a), worin Y eine Methylsulfonyloxy- oder p-Toluolsulfonyloxygruppe ist.

**4.** Verfahren gemäß Anspruch 1, Verfahren (a), oder Anspruch 2, umfassend die Ausführung der Reaktion in Gegenwart von Kaliumjodid in einem Keton-Lösungsmittel.

**5.** Verfahren gemäß Anspruch 1, Verfahren (b), umfassend die Ausführung aller Reaktionsschritte in einem Triisopropylamin-Medium und am Siedepunkt der Reaktionsmischung.

**6.** Verfahren gemäß Anspruch 1 zur Herstellung einer Verbindung der Formel (I) gemäß Anspruch 1, ausgewählt aus der Gruppe, welche umfaßt:
8-[4,4-Bis(4-fluorphenyl)-3-butenyl]-4-hydroxy-4-methyl-2-oxo-1-oxa-3,8-diazaspiro[4,5]decan-hydrochlorid,
8-[4,4-Bis(4-fluorphenyl)-3-butenyl]-3,4-dimethyl-4-hydroxy-2-oxo-1-oxa-3,8-diazaspiro[4,5]decan-hydrochlorid,
8-[4,4-Bis(4-fluorphenyl)-3-butenyl]-3-tert-butyl-4-hydroxy-4-methyl-2-oxo-1-oxa-3,8-diazaspiro[4,5]-decan-hydrochlorid,
8-[4,4-Bis(4-fluorphenyl)-3-butenyl]-3-butyl-4-hydroxy-4-methyl-2-oxo-1-oxa-3,8-diazaspiro[4,5]decan-hydrochlorid,
3-Benzyl-8-[4,4-bis(4-fluorphenyl)-3-butenyl]-4-hydroxy-4-methyl-2-oxo-1-oxa-3,8-diazaspiro[4,5]decan-hydrochlorid,
und deren optische Isomere und Mischungen und alle ihre Solvate, Hydrate, Säureadditions- und quaternären Ammoniumsalze.

**7.** Verfahren zur Herstellung einer pharmazeutischen Zusammensetzung, die als Wirkstoff eine Verbindung der Formel (I) gemäß Anspruch 1 oder ein pharmazeutisch annehmbares Säureadditions- oder quaternäres Ammoniumsalz umfaßt, bei dem der Wirkstoff mit einem pharmazeutisch annehmbaren Träger, Verdünnungsmittel oder Arzneiträger gemischt wird.

**8.** Verwendung einer Verbindung der Formel (I) gemäß Anspruch 1 bei der Herstellung eines Medikaments zur Behandlung von Störungen der Kognitivfunktionen von Säugern.

**Revendications**

**Revendications pour les Etats contractants suivants : AT, BE, CH, DE, DK, FR, GB, IT, LI, LU, NL, SE**

1. Composé de formule (I) :

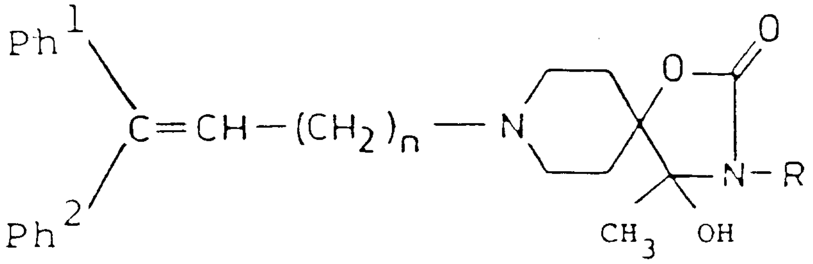

(I)

dans laquelle

R représente un atome d'hydrogène ou un groupe alkyle en $C_1$-$C_{12}$ ou cycloalkyle en $C_3$-$C_6$, ou un groupe aryle en $C_6$-$C_{10}$ carbocyclique ou aryl(en $C_6$-$C_{10}$)alkyle (en $C_1$-$C_4$) carbocyclique, éventuellement substitué sur le noyau aromatique par un ou plusieurs atomes d'halogène, des groupes alkyle en $C_1$-$C_4$ ou alcoxy en $C_1$-$C_4$ ;

$Ph^1$ et $Ph^2$, qui peuvent être identiques ou différents, représentent chacun un groupe phényle non substitué ou substitué par un ou plusieurs atomes d'halogène ou groupes alkyle en $C_1$-$C_4$, alcoxy en $C_1$-$C_4$, trihalogénométhyle ou groupes hydroxyle éventuellement estérifiés par un acide alcanoïque en $C_1$-$C_4$ ; et

n est 1 ou 2,

et isomères optiques et mélanges de ces composés, et tous les solvates, hydrates, sels d'addition d'acides et d'ammonium quaternaire de ces composés.

2. Composé tel que défini dans la revendication 1, choisi dans le groupe comprenant :
le chlorhydrate de 8-[4,4-bis(4-fluorophényl)-3-butényl]-4-hydroxy-4-méthyl-2-oxo-1-oxa-3,8-diazaspiro-[4,5]décane,
le chlorhydrate de 8-[4,4-bis(4-fluorophényl)-3-butényl]-3,4-diméthyl-4-hydroxy-2-oxo-1-oxa-3,8-diazaspiro[4,5]décane,
le chlorhydrate de 8-[4,4-bis(4-fluorophényl)-3-butényl]-3-t-butyl-4-hydroxy-4-méthyl-2-oxo-1-oxa-3,8-diazaspiro[4,5]décane,
le chlorhydrate de 8-[4,4-bis(4-fluorophényl)-3-butényl]-3-butyl-4-hydroxy-4-méthyl-2-oxo-1-oxa-3,8-diazaspiro[4,5]décane,
le chlorhydrate de 8-[4,4-bis(4-fluorophényl)-3-butényl]-4-hydroxy-4-méthyl-2-oxo-1-oxa-3,8-diazaspiro-[4,5]décane,
et les isomères optiques et mélanges de ces composés, et tous les solvates, hydrates, sels d'addition d'acides et d'ammonium quaternaire de ces composés.

3. Composition pharmaceutique, qui comprend, comme substance active, un composé de formule (I) telle que définie dans la revendication 1, ou un de ses sels d'addition d'acide ou d'ammonium quaternaire pharmaceutiquement acceptable, en mélange avec un véhicule, diluant ou excipient pharmaceutiquement acceptable.

4. Procédé de préparation de dérivés 2-oxo-3,8-diazaspiro[4,5]décanes de formule (I) telle que définie dans la revendication 1, qui comprend :

a) la réaction d'un composé de formule (II)

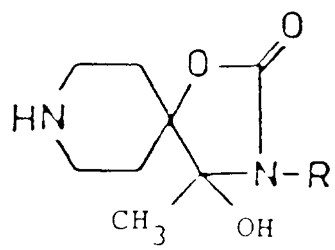

(II)

dans laquelle R est tel que défini dans la revendication 1, avec un composé de formule (III)

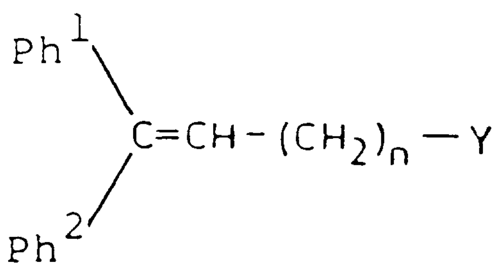

(III)

dans laquelle $Ph^1$, $Ph^2$ et n sont tels que définis dans la revendication 1 et Y représente un atome d'halogène ou un groupe alkyl(en $C_1$-$C_4$)sulfonyloxy ou arylsulfonyloxy ;
ou
b) la réaction d'un composé de formule (VII)

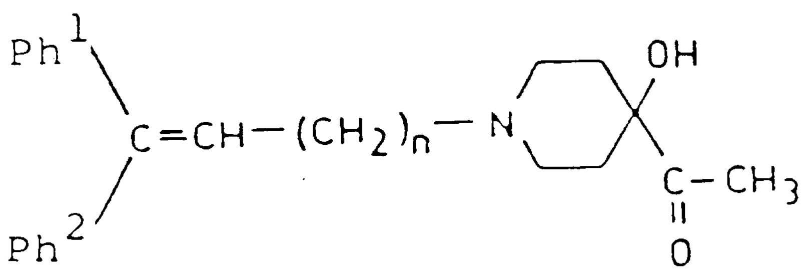

(VII)

dans laquelle $Ph^1$, $Ph^2$ et n sont tels que définis ci-dessus, avec un isocyanate de formule R-NCO, dans laquelle R est tel que défini ci-dessus, autrement qu'un atome d'hydrogène, suivie de la cyclisation du composé résultant de formule (VIII)

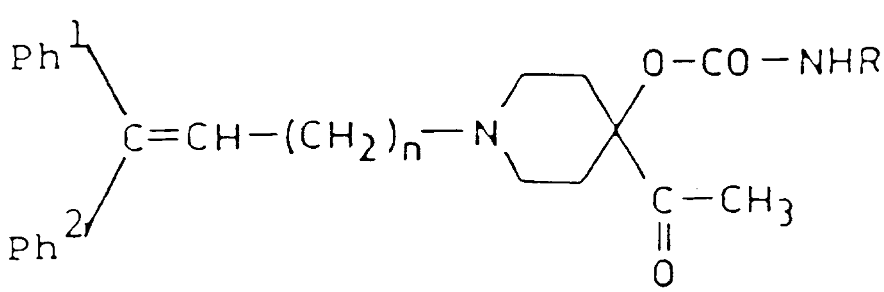

(VIII)

dans laquelle R, $Ph^1$, $Ph^2$ et n sont tels que définis ci-dessus ; ou
c) la cyclisation d'un composé de formule (VIII) telle que définie ci-dessus ; ou

d) la réaction d'un composé de formule (VI)

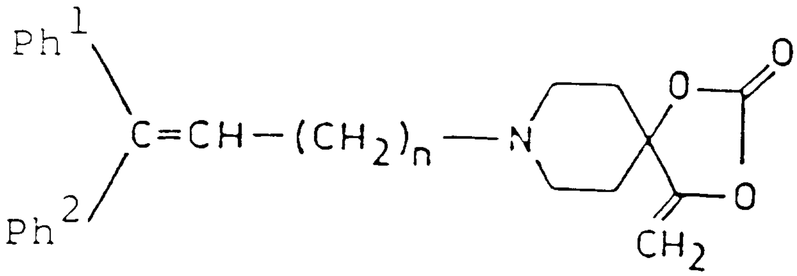

(VI)

dans laquelle $Ph^1$, $Ph^2$ et n sont tels que définis ci-dessus ; ou
e) l'hydratation d'un composé de formule (IV)

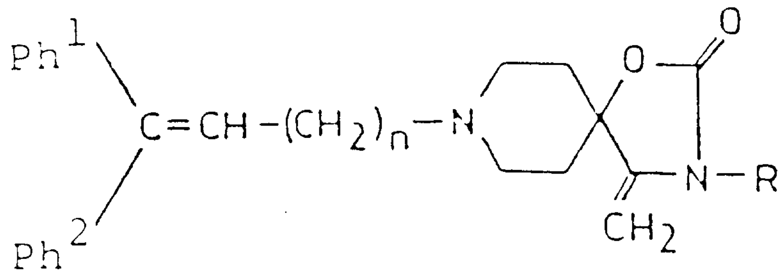

(IV)

dans laquelle R, $Ph^1$, $Ph^2$ et n sont tels que définis ci-dessus, dans un milieu acide ;
suivie, le cas échéant,
de la transformation d'un groupe fonctionnel d'un composé de formule (I), telle que définie ci-dessus, ainsi obtenu en un autre composé de formule (I), telle que définie dans la revendication 1, d'une manière connue ; et/ou
de la réaction d'un composé de formule (I), telle que définie ci-dessus, ainsi obtenu, avec un acide, pour donner un sel d'addition d'acide ; et/ou
du traitement d'un composé de formule (I), telle que définie ci-dessus, obtenu sous forme de sel, avec une base, pour libérer sa forme basique libre ; et/ou
de la transformation d'un composé de formule (I), telle que définie ci-dessus, ainsi obtenu, en son sel d'ammonium quaternaire.

**5.** Procédé selon la revendication 4, procédé a), dans lequel Y est un atome de chlore ou de brome.

**6.** Procédé selon la revendication 4, procédé a), dans lequel Y est un groupe méthylsulfonyloxy ou p-toluènesulfonyloxy.

**7.** Procédé selon la revendication 4 a) ou selon la revendication 5, qui comprend la mise en oeuvre de la réaction en présence de (potassium iodide) iodure de potassium dans un solvant du type cétone.

**8.** Procédé selon la revendication 4, procédé b) qui comprend la mise en oeuvre de toute les étapes réactionnelles dans un milieu triisopropylamine et au point d'ébullition du mélange réactionnel.

**9.** Procédé de préparation d'une composition pharmaceutique telle que définie dans la revendication 3, qui comprend le mélange d'un composé de formule (I) telle que définie dans la revendication 1 ou d'un de ses sels d'addition d'acide ou d'ammonium quaternaire pharmaceutiquement acceptable avec un véhicule, un diluant ou un excipient pharmaceutiquement acceptable.

**10.** Utilisation d'un composé de formule (I) tel que défini dans la revendication 1, pour fabriquer un médicament pour le traitement des troubles des fonctions cognitives chez les mammifères.

**Revendications pour les Etats contractants suivants : ES, GR**

**1.** Procédé de préparation d'un composé de formule (I) :

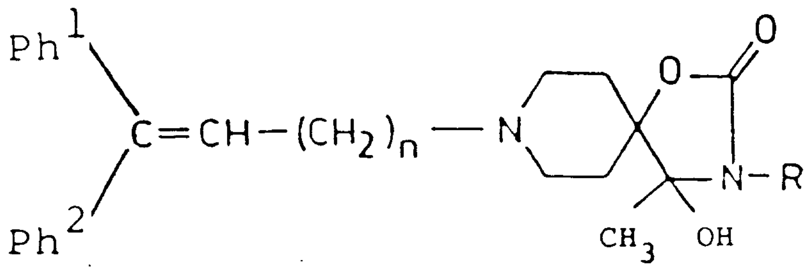

(I)

dans laquelle

R représente un atome d'hydrogène ou un groupe alkyle en $C_1$-$C_{12}$ ou cycloalkyle en $C_3$-$C_6$, ou un groupe aryle en $C_6$-$C_{10}$ carbocyclique ou aryl(en $C_6$-$C_{10}$)alkyle (en $C_1$-$C_4$) carbocyclique, éventuellement substitué sur le noyau aromatique par un ou plusieurs atomes d'halogène, des groupes alkyle en $C_1$-$C_4$ ou alcoxy en $C_1$-$C_4$ ;

$Ph^1$ et $Ph^2$, qui peuvent être identiques ou différents, représentent chacun un groupe phényle non substitué ou substitué par un ou plusieurs atomes d'halogène ou groupes alkyle en $C_1$-$C_4$, alcoxy en $C_1$-$C_4$, trihalogénométhyle ou groupes hydroxyle éventuellement estérifiés par un acide alcanoïque en $C_1$-$C_4$ ; et

n est 1 ou 2,

et d'isomères optiques et de mélanges de ces composés, et de tous les solvates, hydrates, sels d'addition d'acides et d'ammonium quaternaire de ces composés, qui comprend

a) la réaction d'un composé de formule (II)

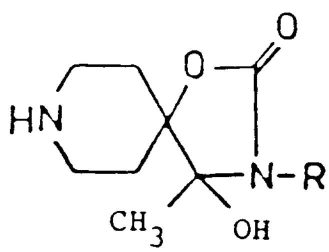

(II)

dans laquelle R est tel que défini dans la revendication 1, avec un composé de formule (III)

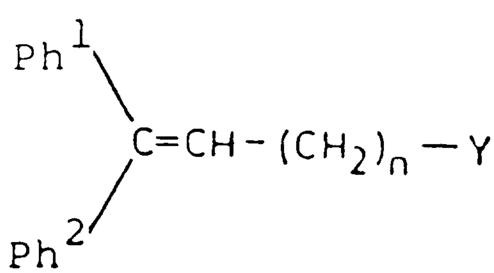

(III)

dans laquelle $Ph^1$, $Ph^2$ et n sont tels que définis ci-dessus et Y représente un atome d'halogène ou un groupe alkyl(en $C_1$-$C_4$)sulfonyloxy ou arylsulfonyloxy ;

ou

b) la réaction d'un composé de formule (VII)

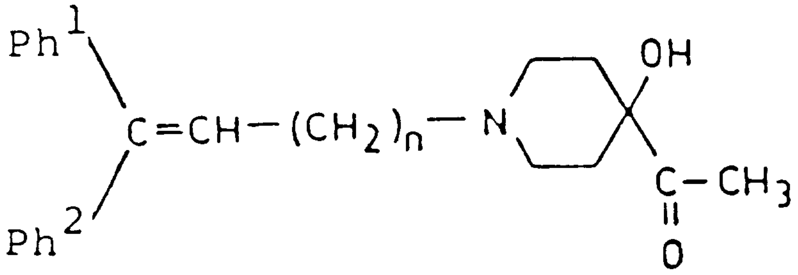

(VII)

dans laquelle $Ph^1$, $Ph^2$ et n sont tels que définis ci-dessus, avec un isocyanate de formule R-NCO, dans laquelle R est tel que défini ci-dessus, autrement qu'un atome d'hydrogène, suivie de la cyclisation du composé résultant de formule (VIII)

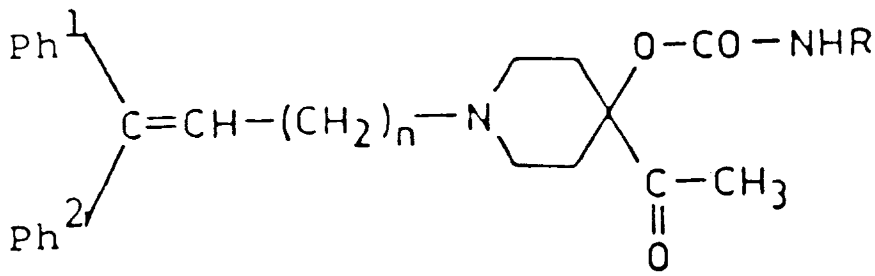

(VIII)

dans laquelle R, $Ph^1$, $Ph^2$ et n sont tels que définis ci-dessus ; ou

c) la cyclisation d'un composé de formule (VIII) telle que définie ci-dessus ; ou

d) la réaction d'un composé de formule (VI)

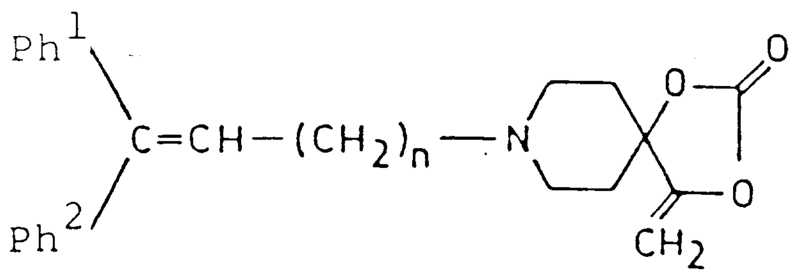

(VI)

dans laquelle $Ph^1$, $Ph^2$ et n sont tels que définis ci-dessus ; ou

e) l'hydratation d'un composé de formule (IV)

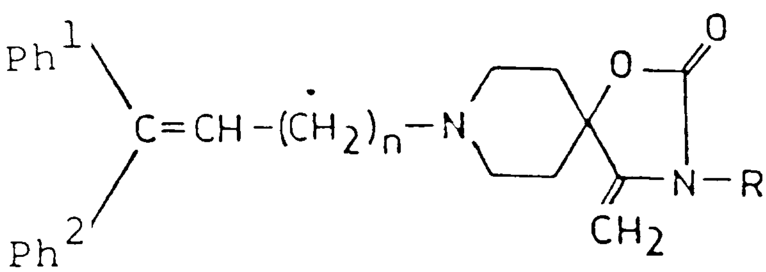

(IV)

dans laquelle R, $Ph^1$, $Ph^2$ et n sont tels que définis ci-dessus, dans un milieu acide ;

suivie, le cas échéant,

de la transformation d'un groupe fonctionnel d'un composé de formule (I), telle que définie ci-dessus, ainsi obtenu en un autre composé de formule (I), telle que définie ci-dessus, d'une manière connue ; et/ou

de la réaction d'un composé de formule (I), telle que définie ci-dessus, ainsi obtenu, avec un acide, pour donner un sel d'addition d'acide ; et/ou

du traitement d'un composé de formule (I), telle que définie ci-dessus, obtenu sous forme de sel, avec une base, pour libérer sa forme basique libre ; et/ou

de la transformation d'un composé de formule (I), telle que définie ci-dessus, ainsi obtenu, en son sel d'ammonium quaternaire.

**2.** Procédé selon la revendication 1, procédé a), dans lequel Y est un atome de chlore ou de brome.

**3.** Procédé selon la revendication 1, procédé a), dans lequel Y est un groupe méthylsulfonyloxy ou p-toluènesulfonyloxy.

**4.** Procédé selon la revendication 1, procédé a), ou selon la revendication 2, qui comprend la mise en oeuvre de la réaction en présence d'iodure de potassium dans un solvant de type cétone.

**5.** Procédé selon la revendication 1, procédé b), qui comprend la mise en oeuvre de toutes les étapes réactionnelles dans un milieu triisopropylamine et au point d'ébullition du mélange réactionnel.

**6.** Procédé selon la revendication 1, pour préparer un composé de formule (I) telle que définie dans la revendication 1, choisi dans le groupe comprenant :

le chlorhydrate de 8-[4,4-bis(4-fluorophényl)-3-butényl]-4-hydroxy-4-méthyl-2-oxo-1-oxa-3,8-diazaspiro-[4,5]décane,

le chlorhydrate de 8-[4,4-bis(4-fluorophényl)-3-butényl]-3,4-diméthyl-4-hydroxy-2-oxo-1-oxa-3,8-diazaspiro[4,5]décane,

le chlorhydrate de 8-[4,4-bis(4-fluorophényl)-3-butényl]-3-t-butyl-4-hydroxy-4-méthyl-2-oxo-1-oxa-3,8-diazaspiro[4,5]décane,

le chlorhydrate de 8-[4,4-bis(4-fluorophényl)-3-butényl]-3-butyl-4-hydroxy-4-méthyl-2-oxo-1-oxa-3,8-diazaspiro[4,5]décane,

le chlorhydrate de 8-[4,4-bis(4-fluorophényl)-3-butényl]-4-hydroxy-4-méthyl-2-oxo-1-oxa-3,8-diazaspiro-[4,5]décane,

et les isomères optiques et mélanges de ces composés, et tous les solvates, hydrates, sels d'addition d'acides et d'ammonium quaternaire de ces composés.

**7.** Procédé de préparation d'une composition pharmaceutique qui comprend, comme substance active, un composé de formule (I) telle que définie dans la revendication 1 ou un de ses sels d'addition d'acide ou d'ammonium quaternaire pharmaceutiquement acceptable, dans lequel la substance active est mélangée avec un véhicule, un diluant ou un excipient pharmaceutiquement acceptable.

**8.** Utilisation d'un composé de formule (I) tel que défini dans la revendication 1, pour fabriquer un médicament pour le traitement des troubles des fonctions cognitives chez les mammifères.